# EUROPÄISCHE PATENTANMELDUNG

(11) **EP 3 061 757 A1**
(43) Veröffentlichungstag der Anmeldung: **31.08.2016**
(21) Anmeldenummer: 16161031.6
(22) Anmeldetag: 31.03.2009
(51) Int. Cl.: C07D 263/04, C07D 295/088, C07D 295/037, C07D 295/145, C07D 295/108, C07C 211/63, C07C 309/06, C07C 311/48, C07F 9/54, C07F 9/6568, H01B 1/12, H01M 10/0567, H01M 10/0568, H01M 10/0569, H01G 11/06, H01G 11/58, H01G 11/60, H01G 11/62, H01G 11/64

(54) **REAKTIVE IONISCHE FLÜSSIGKEITEN**

(30) Priorität: 29.04.2008 DE 102008021271
(62) Teilanmeldung aus: 09737784.0
(71) Anmelder: BASF SE, 67056 Ludwigshafen am Rhein (DE)
(72) Erfinder: SCHMIDT, Michael, 64665 Alsbach-Haehnlein (DE); IGNATYEV, Nikolai, 47058 Duisburg (DE); PITNER, William-Robert, 60599 Frankfurt (DE)
(74) Vertreter: BASF IP Association

(57) **Zusammenfassung**

Die Erfindung betrifft reaktive ionische Flüssigkeiten enthaltend organische Kationen mit elektrochemisch reduktionsanfälligen Gruppen bzw. Substituenten sowie Anionen aus Boraten, Phosphaten und/oder Aluminaten zur Anwendung in elektrochemischen Zellen wie Lithiumionen-Batterien und Doppelschichtkondensatoren oder Lithiumkondensatoren.

## Beschreibung

Die Erfindung betrifft reaktive ionische Flüssigkeiten enthaltend organische Kationen mit elektrochemisch reduktionsanfälligen Gruppen bzw. Substituenten sowie Anionen aus Fluoralkylphosphaten, Fluoralkylphosphinaten, Fluoralkylphosphonaten, Acetaten, Triflaten, Methiden, Boraten, Phosphaten und Aluminaten zur Anwendung in elektrochemischen Zellen wie Lithiumionen-Batterien und Doppelschichtkondensatoren.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf.

Unter "reaktiven ionischen Flüssigkeiten" versteht man ionische Flüssigkeiten, die am organischen Kation elektrochemisch reduktionsanfällige Gruppen bzw. Substituenten wie Cyanogruppen, Estergruppen, Carbonatgruppen oder Seitenketten mit Doppelbindungen aufweisen.

In den letzten Jahren haben ionische Flüssigkeiten mehr und mehr Interesse erlangt und eine Vielzahl von Review-Artikeln haben einzigartige Eigenschaften von Ionischen Fluiden (Ionische Flüssigkeiten, Ionic Liquids bzw. abgekürzt "ILs") beschreiben und verschiedene Einsatzpotentiale aufgezeigt.
Insbesondere erscheinen Ionische Flüssigkeiten viel versprechend für den Einsatz in Energiespeichermedien wie Doppelschichtkondensatoren und Batterien mit einem besonderen Fokus auf Elektro- und Hybridfahrzeugen. Herausragende Eigenschaften von Ionischen Flüssigkeiten beinhalten:
- Eine praktische nicht vorhandene Flüchtigkeit und damit sehr hohe Flammpunkte
- Ein sehr großer Flüssigkeitsbereich über teilweise mehrere 100K
- Eine sehr hohe Polarität und damit in der Regel sehr gute Löslichkeit für anorganische und organische Salze

Während Ionische Flüssigkeiten wie zum Beispiel Ethyl-Methyl-Imidazolium Tetrafluoroborat (EMIBF₄) bereits kommerziell in Doppelschichtkondensatoren (Super- oder Ultracaps) eingesetzt werden, gestaltet sich die Verwendung in Batterien insbesondere Lithium-Ionen-Batterien weiterhin schwierig.

Für Batterieanwendungen wurden folgende ionische Flüssigkeiten beinhaltende, Systeme charakterisiert:
- Ionische Flüssigkeiten in Kombination mit einen Li-Salz enthaltenden Elektrolyten
- Ionische Flüssigkeiten in Kombination mit einen Li-Salz enthaltenden Elektrolyten plus Additivzusätze

Hierbei wurden folgende Ionische Flüssigkeiten verwendet:
- Ionische Flüssigkeiten mit AlCl₄ als Anion (*Generation 0,* sehr frühe Arbeiten)
- Ionische Flüssigkeiten mit Imidazolium basierten Kationen und (per)fluorierten anorganischen oder organischen Anionen (*Generation 1*)
- Ionische Flüssigkeiten mit "Nicht-Imidazolium" basierten Kationen und (per)fluorierten anorganischen oder organischen Anionen (*Generation* 2)

AlCl4- basierte Ionische Flüssigkeiten sind extrem hydrolyseempfindlich und reagieren mit Wasser unter Freisetzung von Salzsäure HCl. Die Entwicklung für Batteriesysteme wurde aufgrund dessen eingestellt. Imidazolium basierte ILs zeigen eine unzureichende reduktive Stabilität und werden daher für die kommerzielle Anwendung in Hochenergiebatterien als nicht viel versprechend angesehen. Ionische Flüssigkeiten mit anorganischen Anionen, insbesondere mit BF4- verursachen, insbesondere in den ersten Lade-/Entladezyklen, einen deutlichen Kapazitätsabfall. Die zurzeit am besten für li-Ionen Batterien geeigneten Ionischen Flüssigkeiten verwenden N,N Di-Alkyl-pyrrollidinium in Kombination mit Bis(trifluormethyl)imiden (siehe z.B. JP 2006-260952).
Allerdings verursachen selbst diese Ionischen Flüssigkeiten noch immer einen signifikanten Rückgang der Leistungsdichten von Lithium-Ionen Batterien.
Grund hierfür ist insbesondere die hohe Viskosität elektrochemisch stabiler Ionischer Flüssigkeiten. Dies führt zu einer deutlich geringeren LithiumIonen-Leitfähigkeit des IL basierten Elektrolyten im Vergleich zu Standard Elektrolytsystemen ohne Ionische Flüssigkeiten (0. Borodin et al., J. of Physical Chemistry B, 2006, 110 (34), p. 16879-16886). Demzufolge zeigen Lithium-Ionen-Batterien mit IL basierten Elektrolyten noch heute eine deutlich geringe Leistungsdichte und Belastbarkeit im Vergleich zu Lithium-Ionen-Batterien mit Standardelektrolyten dementsprechender.
Gerade letzteres ist für Anwendungen in Elektro- und Hybridfahrzeugen als sehr kritisch anzusehen und verhindert-trotz einem Gewinn an Sicherheit - den Einsatz IL basierter Elektrolyte.
Lee et al. (Electrochem. Comm. 8 (2006) 460 konnten zeigen, dass die Verwendung von Imidazolium basierten ILs mit Esterliganden am Stickstoff in Batterieelektrolyten zu einer Verbesserung der Lithiumleitfähigkeit und des Diffusionskoeffizienten von Li-Ionen führt.
Allerdings sind diese Imidazolium-basierten Ionischen Fluide elektrochemisch nicht stabil genug.

Aufgabe der vorliegenden Erfindung war es daher, ionische Flüssigkeiten zu entwickeln, die eine hohe thermische Stabilität, sehr gute Oxidationsstabilität und geringe Korrosivität sowie kostengünstig synthetisierte Anionen aufweisen und die o.g. Nachteile nicht aufweisen.

Die vorliegende Aufgabe wird gelöst durch ionische Flüssigkeiten der allgemeinen Formel I

K⁺A⁻ (I)

worin bedeuten:
K⁺ ein Kation, vorzugsweise reduktionsstabil, ausgewählt aus der Gruppe der allgemeinen Formeln II bis IX

   NR₃-R5 PR₃-R5

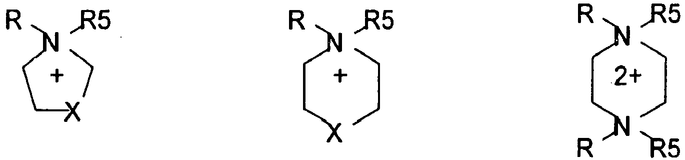

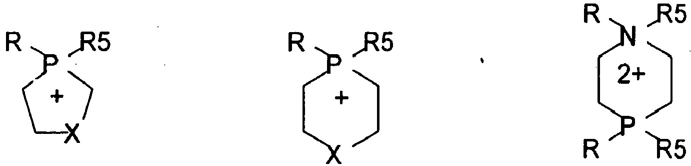

   wobei
   - X: CH₂, O, S oder NR'
   - R': -(CH₂)ₙ-CN, Alkyl C₁ bis C₁₆, vorzugsweise Methyl, Ethyl, Propyl, H
   - R: H, Alkyl C₁ bis C₁₆. vorzugsweise, Methyl, Ethyl, Propyl
   - R5: -(CH₂)ₙ-O-C(O)-R, -(CH₂)ₙ-C(O)-OR, -(CH₂)ₙ-O-C(O)-OR, -(CH₂)ₙ-HC=CH-R oder -(CH₂)ₙ-CN, wobei einzelne der CH₂-Gruppen durch 0, S oder NR ersetzt sind mit n = 1 bis 8
   und
   A⁻ ein Anion ausgewählt aus der Gruppe
      [F_{y}P(CₘF₂ₘ₊₁)_{6-y}]⁻
      (CₘF₂ₘ₊₁)₂P(O)O⁻
      CₘF₂ₘ₊₁P(O)O₂²⁻
      O-C(O)-CₘF₂ₘ₊₁,
      O-S(O)₂-CₘF₂ₘ₊₁
      N(C(O)-CₘF₂ₘ₊₁)₂
      N(S(O)₂-CₘF₂ₘ₊₁)₂
      N(C(O)-CₘF₂ₘ₊₁)(S(O)₂-CₘF₂ₘ₊₁)
      N(C(O)-CₘF₂ₘ₊₁)(C(O)F)
      N(S(O)₂-CₘF₂ₘ₊₁)(S(O)₂F)
      N(S(O)₂F)₂
      C(C(O)-CₘF₂ₘ₊₁)₃
      C(S(O)₂-CₘF₂ₘ₊₁)₃ 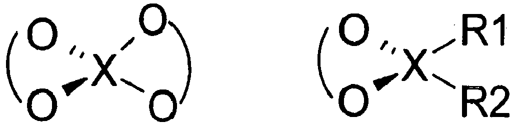 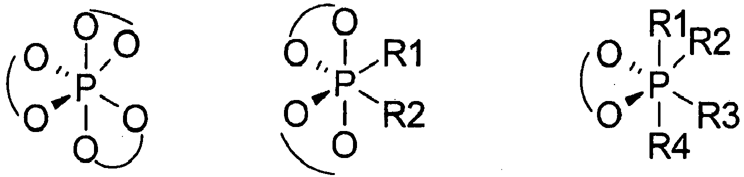
      mit y = 1,2,3,4 oder 5 und m = 1 bis 8, vorzugsweise 1 bis 4 wobei teilweise die CF₂-Gruppen durch 0, S(O)₂, NR oder CH₂ ersetzt sein können und
      wobei 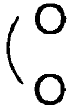 ein 1,2- oder 1,3- Diol, eine 1,2 -oder 1,3- Dicarbonsäure oder 1,2- oder 1,3- Hydroxycarbonsäure
      - X: B oder Al
      - R₁ bis R₄: Halogen, insbesondere F, und/oder ein fluorierter oder nicht fluorierter Alkoxy- oder Carboxy-Rest
      ist.

Gegenüber herkömmlichen ionischen Flüssigkeiten zeichnen sich die erfindungsgemäßen reaktiven ionischen Flüssigkeiten dadurch aus, dass sie am organischen Kation elektrochemisch reduktionsanfällige Gruppen/Substituenten bzw. Seitenketten aufweisen. Dies sind insbesondere
- Cyanogruppen -CN
- Estergruppen -R-C(O)-OR oder -R-O-C(O)-R
- Carbonatfunktionen -R-O-C(O)-OR
- Doppelbindungen in den Seitenketten -R-CH=CH-R

Überraschenderweise bilden die erfindungsgemäßen reaktiven ionischen Flüssigkeiten bei Potentialen zwischen ca. 2 V und 0.9V gegen Li/Li⁺ deutlich eher eine passivierende Deckschicht als Ethylencarbonat (0.7 bis 0.8V gegen Li/Li⁺). Diese Deckschicht ist elektronisch passivierend, aber für Lithium-Ionen durchlässig. Ein weiterer Vorteil besteht in der ausgezeichneten Oxidationsstabilität mit > 5 V gegen Li/Li⁺. Anders als viele andere zur Zeit eingesetzte teilweise hoch toxische (z.B. Propansulton) Additive sind die erfindungsgemäßen reaktiven ionischen Flüssigkeiten nicht flüchtig und besitzen keinen messbaren Dampfdruck.

Vorzugsweise handelt es sich bei den Kationen K⁺ der erfindungsgemäßen ionischen Flüssigkeiten um Kationen ausgewählt aus der allgemeinen Formel IV 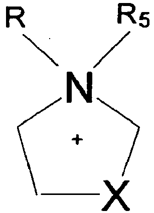 wobei
- X: CH₂, O, S oder NR'
- R': -(CH₂)ₙ-CN, Methyl, Ethyl, Propyl, Butyl, H
- R: H, Methyl, Ethyl, Propyl, Butyl
- R₅: -(CH₂)ₙ-O-C(O)-R, -(CH₂)ₙ-C(O)-OR, -(CH₂)ₙ-O-C(O)-OR, -(CH₂)ₙ-HC=CH-R oder -(CH₂)ₙ-CN, wobei einzelne der CH₂-Gruppen durch 0, S oder NR ersetzt sind mit n = 1 bis 8

Vorzugsweise handelt es sich bei den Anionen A⁻ der erfindungsgemäßen ionischen Flüssigkeiten um folgende Anionen: [F₂P(C₂F₅)₄]⁻, [F₃P(C₂F₅)₃]⁻, [F₄P(C₂F₅)₂]⁻, [F₂P(C₃F₇)₄]⁻, [F₃P(C₃F₇)₃]⁻, [F₄P(C₃F₇)₂]⁻, [F₂P(C₄F₉)₄]⁻, [F₃P(C₄F₉)₃]⁻, [F₄P(C₄F₉)₂]⁻, Perfluoroalkylcarboxylat, Perfluoroalkylsulfonat, Bis(perfluoroalkylsulfonyl)imid, (Perfluoroalkylsulfonyl)(perfluoroalkylcarboxyl)imid, Tris(perfluoroalkylsulfonyl)methid, insbesondere bevorzugt um Trifluoroacetat, Trifluoromethansulfonat (Triflat), Bis(trifluoromethylsulfonyl)imid und Tris(trifluoromethylsulfonyl)methid. Außerdem bevorzugt sind spiro-Oxo-Borate und spiro-Oxo-Phosphate, insbesondere bevorzugt sind spiro-Oxo-Borate.

Ein weiterer Gegenstand ist ein Elektrolyt enthaltend mindestens ein Leitsalz, ein aprotisches Lösungsmittel oder Lösungsmittelgemisch, mindestens eine erfindungsgemäße ionische Flüssigkeit der oben genannten Formel I sowie gegebenenfalls weitere Additive.

In einer bevorzugten Ausführungsform (z.B. bei Verwendung des Elektrolyten in Lithium- oder Lithiumionen-Batterien) ist das Leitsalz ein Lithiumleitsalz wie LiPF₆, LiN(SO₂CF₃)₂. LiN(SO₂C₂F₅)₂, LiF₃P(C₂F₅)₃, LiF₃P(C₄F₉)₃, LiB(C₂O₄)₂ oder LiF₂B(C₂O₄)₂.

In einer weiteren bevorzugten Ausführungsform (z.B. bei Verwendung des Elektrolyten in Doppelschicht- oder Superkondensatoren) ist das Leitsalz eine Verbindung aus der Gruppe N(C₂H₅)₄BF₄ N(C₂H₅)₄PF₆. N(C₂H₅)₃(CH₃) BF₄, N(C₂H₅)₃(CH₃) PF₆, N(C₂H₅)₄N(SO₂CF₃)₂. N(C₂H₅)₃(CH₃) N(SO₂CF₃)₂, N(C₂H₅)₄F₃P(C₂F₅)₃, N(C₂H₅)₃(CH₃)F₃P(C₂F₅)₃

Vorzugsweise besteht das aprotische Lösungsmittel des Elektrolyten aus organischen offenkettigen oder zyklischen Carbonaten, Carbonsäureestern, Nitrilen, Ethern oder Lactonen oder einem Gemisch daraus. Nitrile, insbesondere Acetonitril, werden als Lösungsmittel vorzugsweise in Doppelschichtkondensatoren eingesetzt.

Ein weiterer Gegenstand der vorliegenden Erfindung ist ein Verfahren zur Herstellung von ionischen Flüssigkeiten der Formel I
- Herstellen von heterozyklischen Kationen K⁺ mit Alkyl-, Carboxylat-, Carbönat- oder Cyano-haltigen Seitenketten gemäß Anspruch 1 als Oniumchloride oder -bromide aus den entsprechenden Aminen, Phosphinen, Halogencarboxylaten, Halogencarbonaten, Halogen-Alkylnitrilen oder Alkylhalogeniden nach herkömmlichen nasschemischen Methoden.
- Umsetzung dieser kationischen Oniumchloride oder -bromide mit den entsprechenden anionischen
   - Kalium- und/oder Natrium-Fluoroalkyl-phosphaten oder Kalium und/oder Natrium-Bis(fluoroalkyl)phosphinaten oder Kalium und/oder Natrium-Fluoroalkylphosphonaten oder Fluoroalkylphosphorsäuren oder Bis(fluoroalkyl)phosphinsäuren oder Fluoroalkylphosphonsäuren oder Alkyl- insbesondere Methyl-bis(fluoroalkyl)phosphinaten oder
   - Lithium-Imiden oder -Methiden oder mit Trifluormethan-sulfonsäure oder Kalium- oder Lithium-Trifluoracetaten oder -triflaten oder Alkyl-Triflaten oder Trimethylsilyl-Triflaten oder Trifluormethansulfonsäureanhydrid oder Trifluoracetanhydrid oder
   - Lithium- oder Kalium-Boraten, -Phosphaten oder -Aluminaten im wässrigen und/oder alkoholischen Milieu oder organisches Lösungsmittels oder ohne Lösungsmittel.

Die Herstellung der Kationen sind dem Fachmann bekannt und können nach Verfahren wie sie z.B. in P. Wasserscheid und T. Welton (Eds.) "Ionic Liquids in Synthesis", Wiley -VCH, 2003, S. 7-40 allgemein oder für Imidazolium-Kationen in N. Gathergood, P.J. Scammells, Aust. J. Chem, 55 (2002), Nr. 9, S. 557-560; E. Alcalde, M. Gisbert, L. Perez-Garcia, Heterocycles, 43 (1996(, Nr.3, S. 567-580; Z. Fei, D. Zhao, T. J. Geldbach, R. Scopelliti, P.J. Dyson, Chem. Europ. J., 10 (2004), Nr. 19, S. 4886-4893; D. Liu, Ji. Gui, X. Zhu, L. Song, Z. Sun; Synth. Commun., 37 (2007), Nr.5, S. 759-765; Ya. Peng, F. Yi, G. Song, Yi. Zhang, Monatsh. Chem., 136 (2005), Nr. 10, S. 1751-1755; J. F. Dubreuil, J.P. Bazureau, Tetrahedron Lett., 41 (2000), Nr. 38, S. 7351-7356; S.-K. Fu, Sh.-T. Liu, Synth. Commun., 36 (2006), Nr. 14, S. 2059-2067; M. Yoshizawa, A. Narita, H. Ohno, Aust. J. Chem., 57 (2004), Nr.2, S. 139-144; A. Narita, W. Shibayama, H. Ohno, J. Mater. Chem., 16 (2006), Nr.15, S. 1475-1482; T. Mizumo, E. Marwanta, N. Matsumi, H. Ohno, Chem. Lett., 33 (2004), Nr. 10, S. 1360-1361; D. Zhao, Zh. Fei, T. J. Geldbach, R. Scopelliti, G. Laurenczy, P.J. Dyson, Hel. Chim. Acta., 88 (2005), Nr. 3, S. 665-675; A. Horvath, Synthesis, 1994, S. 102-106; oder für Pyrrolidinium-Kationen in L. Horner, A. Mentrup, Justus Liebigs Ann. Chem. 646 (1961), S. 49-64; Bates et al. , J. Chem. Soc. 1956, S. 388-395, v.Braun Chem. Ber. 70 (1937), S. 983; Z. Dega-Szafran, R. Przybylak, J. Mol. Struct., 436 (1997), Nr. 1, S. 107-122; oder für Piperidinium-Kationen in Walther et al., Chem. Ber., 89 (1956), S 60-65; oder für Morpholinium-Kationen in Gresham et al., J. Am. Chem. Soc., 73 (1951), S. 3168-3171; D. Le Berre, Bull. Soc. Chim. Fr., 1973, S. 2404-2407; O.A. Kazantsev, S.A. Kazakov, K.V. Shirshin, S.M. Danov, Russ. J. Org. Chem., 36 (2000), Nr. 3, S. 343-349; oder für Piperazinium-Kationen in Z. Dega-Szafran, M. Jaskolski, I. Kurzyca, P. Barczynski, M. Szafran, J. Mol. Struct, 614 (2002), Nr.1-3, S. 23-32 beschrieben sind, hergestellt werden.

Die erfindungsgemäßen Anionen sind oxidationsstabil, wie aus den Tabellen 1.1 bis 1.3 zu entnehmen ist.

**Tabelle 1.1:**

| Elektrochemische Stabilität von reaktiven ionischen Flüssigkeiten mit Tris(pentafluoroethyl)trifluorophosphat Anion (FAP-Anion) und Bis(pentafluoroethyl)phosphinat Anion, (C₂F₅)₂P(O)O⁻, in Vergleich zu ähnlichen ionischen Flüssigkeiten mit Triflat und Tetrafluoroborat Anionen | | | |
|---|---|---|---|
| Reaktive ionische Flüssigkeit | E_{(Ox)}, V | E_{(Red)}, V | Elektrochem. Fenster |
| 1-Ethyl-3-methyl-imidazolium FAP | 3,9 | -2,5 | 6,4 |
| 1-Ethyl-3-methyl-imidazolium (C₂F₅)₂P(O)O⁻ | 3,6 | -2,6 | 6,2 |
| 1-Ethyl-3-methyl-imidazolium Triflat | 2,8 | -2,5 | 5,3 |
| 1-Ethyl-3-methylimidazo-lium BF₄⁻ | 2,6 | -2,6 | 5,2 |

**Tabelle 1.2:**

| Elektrochemische Stabilität von reaktiven ionischen Flüssigkeiten mit Bis(trifluoromethylsufonyl)imid Anion und Triflat Anion im Vergleich zur ionischen Flüssigkeit mit Tetrafluoroborat Anion | | | |
|---|---|---|---|
| Reaktive ionische Flüssigkeit | E_{(Ox)}, V | E_{(Red)}, V | Elektrochem. Fenster |
| 1-Butyl-1-methylpyrrolidinium Bis(trifluoromethylsulfonyl)imid | -3,5 | -3,3 | 6,8 |
| T rihexyl(tetradecyl)phosphonium Bis(trifluoromethylsulfonyl) imid | | -3,4 | 6,5 |
| 1-Hexyl-3-methylimidazolium Bis(trifluoromethylsulfonyl)imid | 3,7 | -2,6 | 6,3 |
| 1-Ethyl-3-methylimidazolium Triflat | 2,8 | -2,5 | 5,3 |
| 1-Ethyl-3-methylimidazolium BF₄⁻ | 2,6 | -2,6 | 5,2 |

**Tabelle 1.3:**

| Elektrochemische Stabilität von ionischen Flüssigkeiten mit Borat-, Phosphat- und Aluminat-Anionen | |
|---|---|
| | E(_{Ox}), vs. Li/Li⁺ |
| 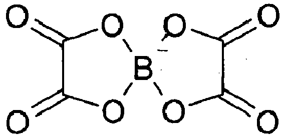 | 4,7- 4,8 V |
| Bis Oxalato Borat (BOB) | |
| 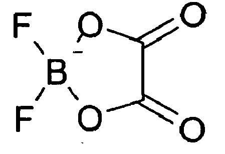 | 4,8- 5,0 V |
| Di-Fluoro Oxalato Borat (DFOB) | |
| 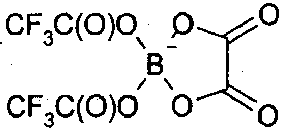 | 4,8- 5,0 V |
| Di-Trifluoracetato Oxalato Borat (D(Ac)OB) | |
| 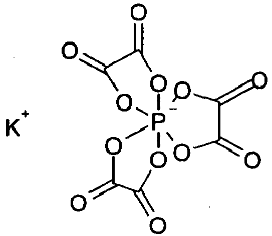 | 4,7-4,8 V |
| Kalium Tris Oxalato Phosphat (KTOP) | |
| 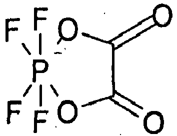 | 4,8-5,0 V |
| Tetra-Fluor Oxalato Phosphat (TFOP) | |
| 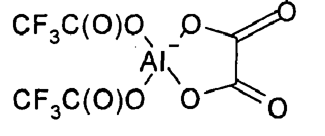 | > 5V |
| Di-(Trifluoracetato) Oxalato Aluminat (D(Ac)OAl) | |

Die Herstellung der Fluoralkylphosphat-basierten reaktiven ionischen Flüssigkeiten geschieht durch Umsetzung der entsprechenden kationischen Onium-Chloride oder Onium-Bromide mit den entsprechenden Kalium oder Natrium-Fluoroalkylphosphaten oder Fluoroalkylphosphorsaure (HFAP) im wässrigen Milieu.

Die Herstellung der Bis(fluoroalkyl)phosphinat-basierten Ionischen Fluide geschieht nach drei verschiedenen Methoden:
- Herstellung dieser Ionischen Fluide mittels Phosphinsäure (siehe Beispiel 5.1)
- Herstellung dieser Ionischen Fluide über die entsprechenden Kaliumsalze (wie Kalium-fluoroalkylphosphinat) (siehe Beispiel 5.2)
- Herstellung dieser Ionischen Fluide über die entsprechenden Alkyl-Bis(fluoroalkyl)phosphinate, vorzugsweise Methyl-Phosphinate (siehe Beispiel 5.3)

Dabei ist die Herstellung der Bis(fluoroalkyl)phosphinat-basierten Ionischen Flüssigkeiten über die Phosphinsäure und Methyl-Phosphinate bevorzugt.

Die Herstellung der Imid- und Methid-basierten ionischen Flüssigkeiten geschieht durch Umsetzung der entsprechenden, kationischen Onium-Chloride oder Onium-Bromide mit den entsprechenden Lithiumsalzen im wässrigen Milieu.

Die Herstellung der Trifluoroacetat- und Triflat-basierten ionischen Flüssigkeiten geschieht nach drei verschiedenen Methoden:
- Herstellung der Triflat-basierten ionischen Fluide mittels Triflic-Säure (siehe Beispiel 5.1)
- Herstellung der ionischen Fluide über die entsprechenden Kalium- oder Lithium-Trifluoroacetate oder -Triflate (siehe Beispiel 5.2)
- Herstellung der ionischen Fluide über die entsprechenden Methyl-Triflate, Trimethylsilyl-Triflate, Triflic-Anhydride oder Trifluoracet-anhydride (siehe Beispiel 5.3)

Dabei ist die Herstellung der Trifluoracetat- und Triflat-basierten ionischen Flüssigkeiten über die Triflic-Säure oder Trifluoressigsäure und/oder Methyl- oder Ethyl-Triflate oder Trifluoroacetate oder Trimethylsilyl-Triflate, Triflic-Anhydride oder Trifluoracet-Anhydride Methode bevorzugt.

Die Herstellung der Borat-, Phosphat- oder Aluminat-basierten reaktiven ionischen Flüssigkeiten geschieht durch Umsetzung der entsprechenden, kationischen Onium-Chloride oder Onium-Bromide mit den entsprechenden anionischen Kalium- oder Lithium-Boraten, -Phosphaten oder -Aluminaten im wässrigen oder organischen Milieu.

Die Umsetzung der erfindungsgemäßen Kationen mit den Anionen zum Endprodukt kann bei Temperaturen von 0 bis 150°C, vorzugsweise bei 0 bis 50°C und insbesondere bei Raumtemperatur erfolgen.

Geeignete Lösungsmittel oder Lösungsmittelgemische sind Wasser bzw. VE-Wasser, Alkohole, Dioxan, Acetonitril und Aceton. Als Alkohol wird bevorzugt Methanol und iso-Propanol eingesetzt. Im Fall der Anwendung von Methyl-Phosphinaten braucht man üblicherweise kein Lösungsmittel.

Ein weiterer Gegenstand der vorliegenden Erfindung ist eine elektrochemische und/oder elektrooptische Vorrichtung enthaltend mindestens einen Elektrolyten, welcher mindestens eine ionische Flüssigkeit der allgemeinen Formel I enthält. Vorzugsweise kann die Vorrichtung eine Solarzelle, Lithium- oder Lithiumionenbatterie, ein Doppelschicht- und Superkondensator, Lithium-Kondensator, eine Licht - emittierende Vorrichtung, ein elektrochemischer Sensor und/ oder Biosensor sein.

Weiterhin ist Gegenstand der vorliegenden Erfindung die Verwendung der genannten reaktiven ionischen Flüssigkeiten gemäß der allgemeinen Formel I als Leitsalz oder Additiv in Elektrolyten für elektrochemische oder elektrooptische Zellen.

In einer weiteren bevorzugten Ausführungsform werden die erfindungsgemäßen reaktiven ionischen Flüssigkeiten als Leitsalz oder Additiv in Elektrolyten für Batterien, sekundären Lithiumbatterien, Doppelschicht- und Superkondensatoren oder Lithium-Kondensatoren verwendet.

Weiterhin ist Gegenstand der vorliegenden Erfindung die Verwendung der erfindungsgemäßen Elektrolyten in elektrochemischen und/oder elektrooptischen Vorrichtungen. Vorzugsweise handelt es bei diesen Vorrichtungen um Lithium- oder Lithiumionen-Batterien sowie Doppelschicht- und Superkondensatoren oder Lithium-Kondensatoren.

Die folgenden Beispiele sollen die vorliegende Erfindung verdeutlichen. Sie sind jedoch keinesfalls als limitierend zu betrachten. Alle Verbindungen oder Komponenten, die in den Zubereitungen verwendet werden können, sind entweder bekannt und käuflich erhältlich oder können nach bekannten Methoden synthetisiert werden. Die in den Beispielen angegebenen Temperaturen gelten immer in °C. Es versteht sich weiterhin von selbst, dass sich sowohl in der Beschreibung als auch in den Beispielen die zugegebenen Mengen der Komponenten in den Zusammensetzungen immer zu insgesamt 100% addieren. Gegebene Prozentangaben sind immer im gegebenen Zusammenhang zu sehen. Sie beziehen sich üblicherweise aber immer auf die Masse der angegebenen Teil- oder Gesamtmenge.

### Beispiele

### Herstellung der Kationen

### Beispiel 1: Herstellung von heterozyklischen Kationen mit Allyl-Seitenketten

### Allgemeine Vorschrift:

Zu 1 mol des entsprechenden Amins oder Phosphin werden 1,1mol eines Allylchlorids tropfenweise zugegeben. Hierbei ist darauf zu achten, dass die Temperatur zwischen 30-35°C gehalten wird. Die Reaktionsmischung wird danach 3h bis 48 h bei 40°C bis 50° C (im Falle der Bildung von festen Produkten wird die Reaktionsmischung mit Dichlormethan oder Acetonitril verdünnt) gerührt und das überschüssige Allylchlorid und Lösungsmittel danach im Vakuum (2·10⁻³ mbar) abdestilliert. Die Produktausbeute ist nahe zu quantitativ.

**Tab. 1**

| **Eingesetztes Amin/Phospin** | **Eingesetztes Allylchlorid** | **Produkt** |
|---|---|---|
| N(C₂H₅)₃ | Cl-CH₂ -CH=CH₂ | [N(C₂H₅)₃(CH₂-CH=CH₂)] Cl |
| N(CH₃)(C₂H₅)₂ | Cl-CH₂ -CH=CH₂ | [N(CH₃)(C₂H₅)₃(CH₂-CH=CH₂)]Cl |
| P(C₄H₉)₃ | Cl-CH₂ -CH=CH₂ | [P(C₄H₉)₃(CH₂-CH=CH₂)] Cl |
| 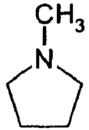 | Cl-CH₂ -CH=CH₂ | 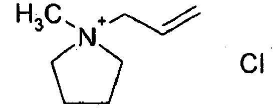 |
| 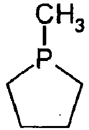 | O-CH₂ -CH=CH₂ | 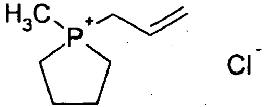 |
| 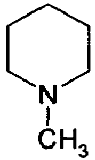 | Cl-CH₂ -CH=CH₂ | 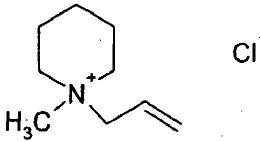 |
| 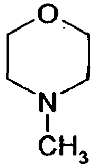 | O-CH₂ -CH=CH₂ | 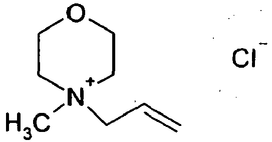 |
| 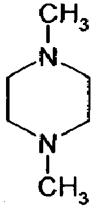 | Cl-CH₂ -CH=CH₂ | 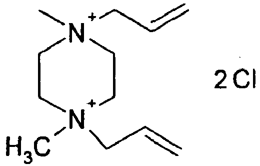 |
| 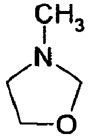 | Cl-CH₂ -CH=CH₂ | 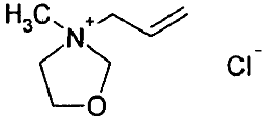 |

### Beispiel 2: Herstellung von heterozyklischen Kationen mit Carboxylat-Seitenketten bzw. Carbonat-Seitenketten

Allgemeine Vorschrift:
In einem 2 L Mehrhalsrundkolben mit KPG-Rührer werden 1 Mol des entsprechenden Amins oder Phosphins in 300 ml Acetonitril vorgelegt und auf 80 °C gebracht. Anschließend werden 1,1 Mol des entsprechenden Halogencarboxylats bzw., -carbonats (vorzugsweise Bromcarboxylat bzw. - Carbonat) langsam innerhalb von1,5 h hinzugetropft.

Danach wurde die Reaktionsgemisch weiterhin für 0.5 bis 48 Stunden bei dieser Temperatur reagieren lassen und in 1 L Ethylacetat eingerührt, wobei das Produkt als weißer Feststoff ausfällt. Das Produkt wird abgesaugt, mit Ethylacetat nachgewaschen und getrocknet (Rotationsverdampfer bei ca. 30°C Wasserbad). Die Ausbeute beträgt zwischen 90 und 95%

**Tab. 2**

| **Eingesetztes Amin/Phospin** | **Chlor (Brom) -Alkyl-Carboxylat** | **Produkt** |
|---|---|---|
| N(C₂H₅)₃ | Br-CH₂-C(O)-O-C₂H₅ | [N(C₂H₅)₃(CH₂-C(O)-O-C₂H₅)] Br |
| N(CH₃)₂(C₂H₅) | Br-CH₂-CH₂-C(O)-O-CH₃ | [N(CH₃)₂(C₂H₅) (CH₂-CH₂-C(O)-O-CH₃)] Br |
| N(CH₃)₂(C₂H₅) | Br -CH₂-C(O)-CH₂-C(O)-O-CH₃ | [N(CH₃)₂(C₂H₅) (CH₂-C(O)-CH₂-C(O)-O-CH₃)] Br |
| N(CH₃)₂(C₂H₅) | Br-CH₂-O-C(O)-O-CH₃ | [N(CH₃)₂(C₂H₅)(CH₂-O-C(O)-O-CH₃)] Br |
| P(C₄H₉)₃ | Br -CH₂-C(O)-O-C₂H₅ | [P(C₄H₉)₃(CH₂-C(O)-O-C₂H₅)] Br |
| 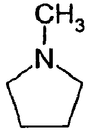 | Br -CH₂-CH₂-C(O)-O-CH₃ | 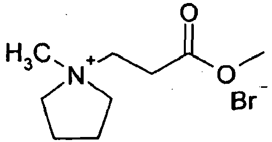 |
| 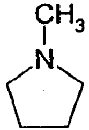 | Br -CH₂-C(O)-CH₂-C(O)-O-CH₃ | 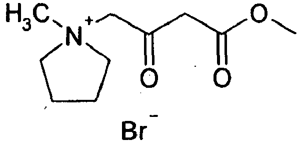 |
| 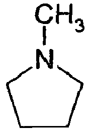 | Br-CH₂-O-C(O)-O-CH₃ | 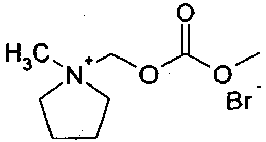 |
| 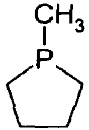 | Br -CH₂-CH₂-C(O)-O-CH₃ | 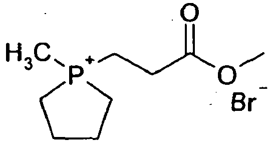 |
| 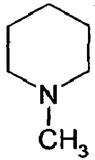 | Br -CH₂-CH₂-C(O)-O-CH₃ | 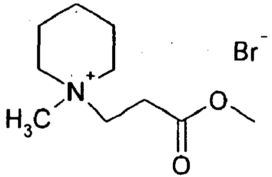 |
| 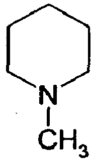 | Cl-CH₂-O-C(O)-O-CH₃ | 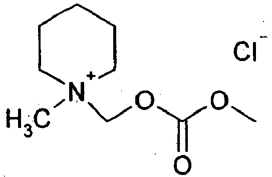 |
| 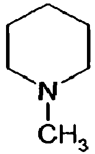 | Cl-CH₂-C(O)-CH₂-C(O)-O-CH₃ | 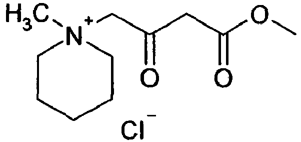 |
| 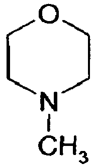 | Cl-CH₂-CH₂-C(O)-O-CH₃ | 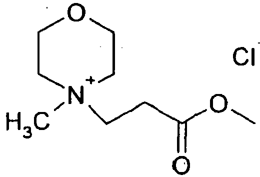 |
| 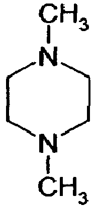 | Cl-CH₂-CH₂-C(O)-O-CH₃ | 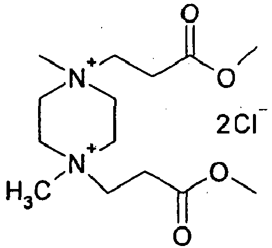 |
| 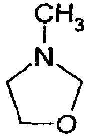 | Cl-CH₂-CH₂-C(O)-O-CH₃ | 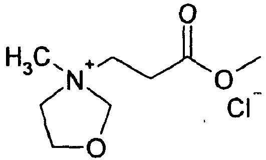 |

### Beispiel 3: Herstellung von heterozyklischen Kationen mit Cyano-haltigen Seitenketten

### Allgemeine Vorschrift:

Zu 1 mol des entsprechenden Amins oder Phosphin werden 1,1mol eines Chlor-Alkyl-Nitrils (bzw. Brom- oder Iod-Alkyl-Nitrils) tropfenweise zugegeben. Hierbei ist darauf zu achten, dass die Temperatur zwischen 30-50° gehalten wird. Die Reaktionsmischung wird danach 3h bis 48 h bei 50°C bis 80° C (im Falle der Bildung von festen Produkten wird die Reaktionsmischung mit Acetonitril verdünnt) gerührt und das überschüssige Chlor-Alkyl-Nitril und Lösungsmittel danach im Vakuum (2·10⁻³ mbar) abdestilliert. Die Produktausbeute ist nahe zu quantitativ.

**Tab. 3**

| **Eingesetztes Amin/Phospin** | **Chlor-Alkyl-Nitril** | **Produkt** |
|---|---|---|
| N(C₂H₅)₃ | Cl-CH₂-CH₂-CN | [N(C₂H₅)₃(CH₂-CH₂-CN)] Cl |
| N(CH₃)₂(C₂H₅) | Cl-CH₂-CH₂-CN | [N(CH₃)₂(C₂H₅)(CH₂-CH₂-CN)]Cl |
| P(C₄H₉)₃ | Cl-CH₂-CH₂-CN | [P(C₄H₉)₃(CH₂-CH₂-CN)]Cl |
| 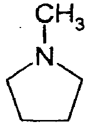 | Cl-CH₂-CH₂-CN | 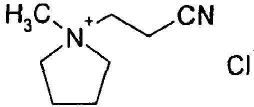 |
| 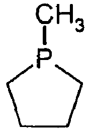 | Cl-CH₂-CH₂-CN | 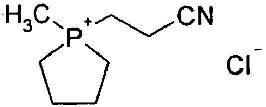 |
| 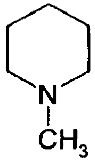 | Cl-CH₂-CH₂-CN | 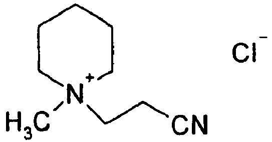 |
| 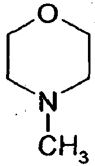 | Cl-CH₂-CH₂-CN | 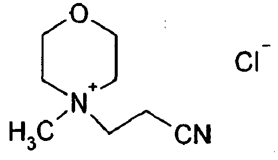 |
| 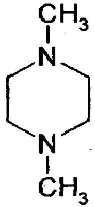 | Cl-CH₂-CH₂-CN | 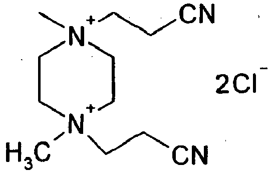 |
| 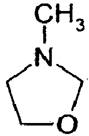 | O-CH₂-CH₂-CN | 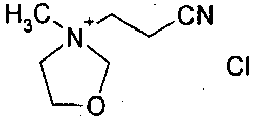 |

### Herstellung der Fluoralkylphosphat- und Fluoralkylphosphinat-basierten reaktiven ionischen Flüssigkeiten

### Beispiel 4: Herstellung der Fluoroalkylphosphat-basierten reaktiven ionischen Flüssigkeiten

### Allgemeine Vorschrift

In einem Kolben mit Magnetfisch werden 1 mol des entsprechenden Onium-Chlorids oder Onium-Bromids (aus den vorher beschriebenen Beispielen) in 200 ml VE-Wasser gelöst und anschließend langsam mit 1 Mol des entsprechenden Kalium (oder Natrium) -Fluoroalkylphosphat versetzt. Es bilden sich unmittelbar 2 Phasen.

Diese zwei Phasen werden noch für 1 h bei Raumtemperatur nachgerührt. Die organische Phase wird danach abgetrennt und dann noch 5 mal mit je 100 ml VE-Wasser Chlorid frei gewaschen (Nachweis: 1 molare Silbernitratlösung) und im Vakuum bei 80° C - 90° C getrocknet.

### Beispiel 5.1: Herstellung der Bis(fluoroalkyl)phosphinat-basierten reaktiven ionischen Flüssigkeiten mittels Phosphinsäure

### Allgemeine Vorschrift

In einem Kolben mit Magnetfisch werden 1 mol des entsprechenden Onium-Chlorids (aus den vorher beschriebenen Beispielen) in 200 ml VE-Wasser gelöst und anschließend langsam mit 1 Mol des entsprechenden Bis(fluoroalkyl)phosphinsäure versetzt.

Die Reaktionsmischung wird noch für 1 h bei Raumtemperatur nachgerührt und Wasser zusammen mit entstehender Salzsäure abdestilliert. Um vollständige Abtrennung von Salzsäure zu erreichen, kann man die mehrmalige azeotropische Destillation mit Dioxan und Wasser benutzen (bis zum negativen Test mit Silbernitratlösung). Nach der Trocknung im Vakuum bei 80° C - 90° C erhält man die Bis(fluoroalkyl)phosphinate in nahe zu quantitativer Ausbeute.

### Beispiel 5.2: Herstellung der Bis(fluoroalkyl)phosphinat-basierten reaktiven ionischen Flüssigkeiten über die entsprechenden Kaliumsalze

### Allgemeine Vorschrift

In einem Kolben mit Magnetfisch werden 1 mol des entsprechenden Onium-Chlorids (aus den vorher beschriebenen Beispielen) in *iso-*Propanol (oder Methanol, oder Acetonitril) gelöst und anschließend langsam mit 1 Mol des entsprechenden Kalium-Fluoroalkylphosphinat versetzt.

Die Reaktionsmischung wird noch für 1 h bei Raumtemperatur nachgerührt und entstehender KCl abfiltriert. Nach der Entfernung von *iso-*Propanol (oder Methanol, oder Acetonitril) im Vakuum mit Hilfe von Rotationsverdampfer erhält man die Bis(fluoroalkyl)phosphinate in nahe zu quantitativer Ausbeute.

### Beispiel 5.3: Herstellung der Bis(fluoroalkyl)phosphinat-basierten reaktiven ionischen Flüssigkeiten über die entsprechenden Methylphosphinate

In einem Kolben mit Magnetfisch werden 1 mol des entsprechenden Onium-Chlorids (oder Bromids) (aus den vorher beschriebenen Beispielen) mit 1 bis 1,1 Mol des entsprechenden Methyl -bis(fluoroalkyl)phosphinat versetzt.

Die Reaktionsmischung wird für 1 bis 20 h bei Raumtemperatur oder bei Heizung bis 100° C nachgerührt und entstehender CH₃Cl (oder CH₃Br) und Überschuss von Methyl Bis(fluoroalkyl)phosphinat im Vakuum entfernt. Bis(fluoroalkyl)phosphinate bilden sich in nahe zu quantitativer Ausbeute.

**Tab.4**

| **Eingesetztes Oniumsalz** | **Eingesetztes Kaliumsalz oder Phospinsaure oder Methylphosphinate** | **Ionische Flüssigkeit** |
|---|---|---|
| [N(C₂H₅)₃(CH₂-CH=CH₂)]Cl | K F₃P(C₂F₅)₃ | [N(C₂H₅)₃(CH₂-CH=CH₂)] F₃P(C₂F₅)₃ |
| [N(C₂H₅)₃(CH₂-CH=CH₂)]Cl | K F₃P(C₄F₉)₃ | [N(C₂H₅)₃(CH₂-CH=CH₂)] F₃P(C₄F₉)₃ |
| [N(C₂H₅)₃(CH₂-CH=CH₂)]Cl | K OP(O)(C₂F₅)₂ | [N(C₂H₅)₃(CH₂-CH=CH₂)] OP(O)(C₂F₅)₂ |
| | HOP(O)(C₂F₅)₂ | |
| | CH₃OP(O)(C₂F₅)₂ | |
| [N(C₂H₅)₃(CH₂-CH=CH₂)] Cl | K OP(O)(C₄F₉)₂ | [N(C₂H₅)₃(CH2-CH=CH₂)] OP(O)(C₄F₉)₂ |
| | HOP(O)(C₄F₉)₂ | |
| | CH₃OP(O)(C₄F₉)₂ | |
| [N(CH₃)(C₂H₅)₃(CH₂-CH=CH₂)]Cl | K F₃P(C₂F₅)₃ | [N(CH₃)(C₂H₅)₃(CH₂-CH=CH₂)] F₃P(C₂F₅)₃ |
| [P(C₄H₉)₃(CH₂-CH=CH₂)]Cl | K F₃P(C₂F₅)₃ | [P(C₄H₉)₃(CH₂-CH=CH₂)] F₃P(C₂F₅)₃ |
| 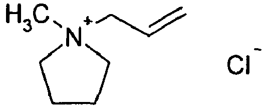 | K F₃P(C₂F₅)₃ | 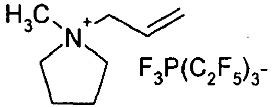 |
| 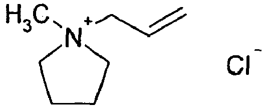 | K F₃P(C₄F₉)₃ | 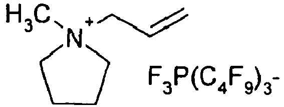 |
| 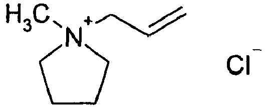 | K OP(O)(C₂F₅)] | 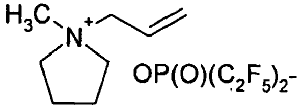 |
| | HOP(O)(C₂F₅)₂ | |
| | CH₃OP(O)(C₂F₅)₂ | |
| 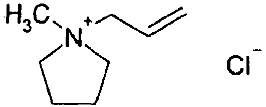 | K OP(O)(C₄F₉)₂ | 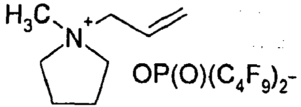 |
| | HOP(O)(C₄F₉)₂ | |
| | CH₃OP(O)(C₄F₉)₂ | |
| 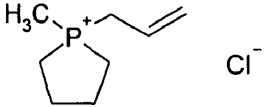 | K F₃P(C₂F₅)₃ | 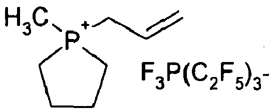 |
| 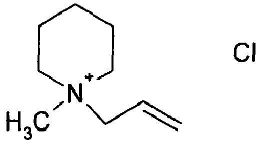 | K F₃P(C₂F₅)₃ | 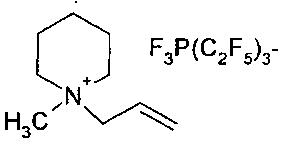 |
| 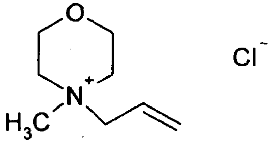 | K F₃P(C₂F₅)₃ | 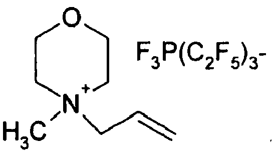 |

**Tab. 5**

| **Eingesetztes Oniumsalz** | **Eingesetztes Kaliumsalz oder Methylphosphinate** | **Ionisches Fluid** |
|---|---|---|
| [N(C₂H₅)₃(CH₂-C(O)-O-C₂H₅)] Br | K F₃P(C₂F₅)₃ | [N(C₂H₅)₃(CH₂-C(O)-O-C₂H₅)] F₃P(C₂F₅)₃ |
| [N(C₂H₅)₃(CH₂-C(O)-O-C₂H₅)] Br | K F₃P(C₄F₉)₃ | [N(C₂H₅)₃(CH₂-C(O)-O-C₂H₅)] F₃P(C₄F₉)₃ |
| [N(C₂H₅)₃(CH₂-C(O)-O-C₂H₅)] Br | K OP(O)(C₂F₅)₂ | [N(C₂H₅)₃(CH₂-C(O)-O-C₂H₅)]OP(O)(C₂F₅)₂ |
| [N(C₂H₅)₃(CH₂-C(O)-O-C₂H₅)] Br | K OP(O)(C₄F₉)₂ | [N(C₂H₅)₃(CH₂-C(O)-O-C₂H₅)]OP(O)(C₄F₉)₂ |
| | CH₃OP(O)(C₄F₉)₂ | |
| [N(CH₃)₂(C₂H₅)(CH₂-CH₂-C(O)-O-CH₃)] Br | K F₃P(C₂F₅)₃ | [N(CH₃)₂(C₂H₅) (CH₂-CH₂-C(O)-O-CH₃)] F₃P(C₂F₅)₃ |
| [N(CH₃)₂(C₂H₅) (CH₂-C(O)-CH₂-C(O)-O-CH₃)] Br | K F₃P(C₂F₅)₃ | [N(CH₃)₂(C₂H₅)(CH₂-C(O)-CH₂-C(O)-O-CH₃)] F₃P(C₂F₅)₃ |
| [N(CH₃)₂(C₂H₅) (CH₂-O-C(O)-O-CH₃)] Br | K F₃P(C₂F₅)₃ | [N(CH₃)₂(C₂H₅) (CH₂-O-C(O)-O-CH₃)] F₃P(C₂F₅)₃ |
| [P(C₄H₉)₃(CH₂-C(O)-O-C₂H₅)] Br | K F₃P(C₂F₅)₃ | [P(C₄H₉)₃(CH₂-C(O)-O-C₂H₅)] F₃P(C₂F₅)₃ |
| 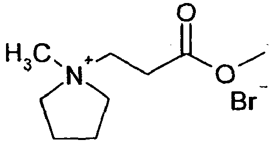 | K F₃P(C₂F₅)₃ | 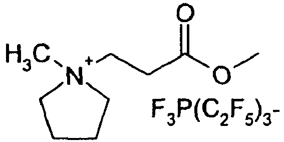 |
| 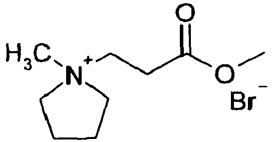 | K F₃P(C₄F₉)₃ | 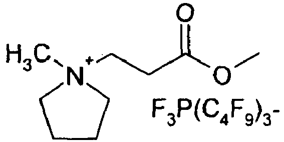 |
| 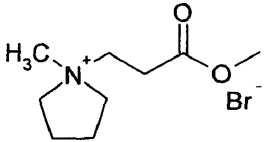 | K OP(O)(C₂F₅)₂ | 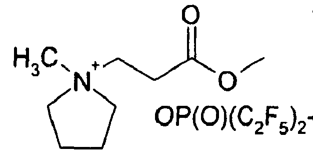 |
| | CH₃OP(O)(C₂F₅)₂ | |
| 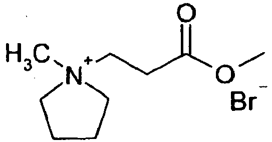 | K OP(O)(C₄F₉)₂ | 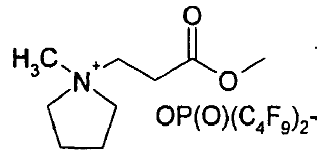 |
| | CH₃OP(O)(C₄F₉)₂ | |
| 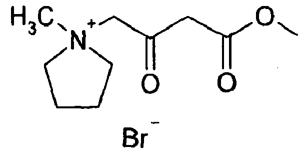 | K F₃P(C₂F₅)₃ | 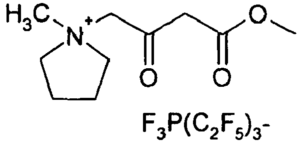 |
| 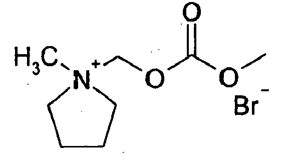 | K F₃P(C₂F₅)₃ | 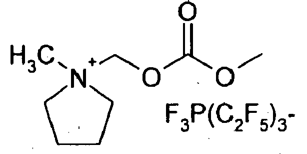 |
| 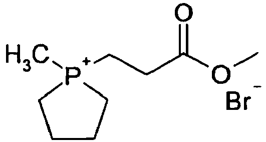 | K F₃P(C₂F₅)₃ | 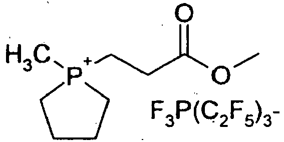 |
| 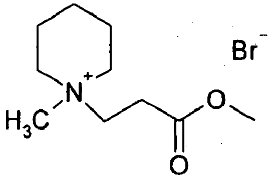 | K F₃P(C₂F₅)₃ | 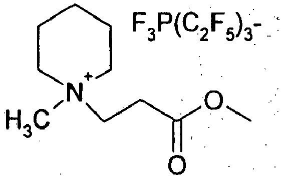 |
| 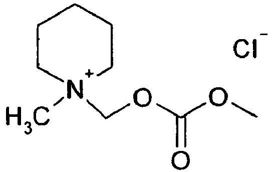 | K F₃P(C₂F₅)₃ | 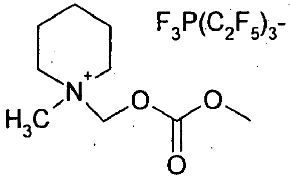 |
| 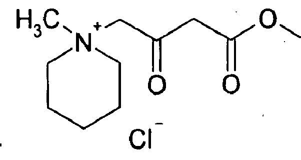 | K F₃P(C₂F₅)₃ | 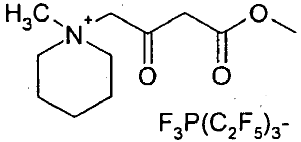 |
| 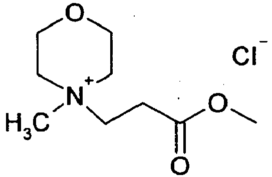 | K F₃P(C₂F₅)₃ | 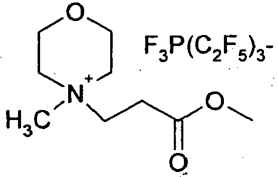 |
| 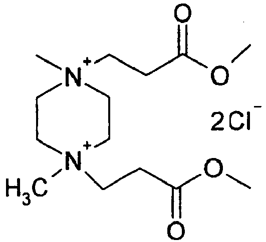 | K F₃P(C₂F₅)₃ | 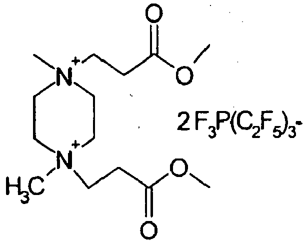 |
| 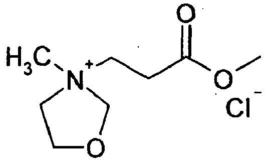 | K F₃P(C₂F₅)₃ | 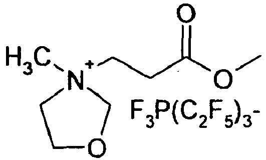 |

**Tab.6**

| **Eingesetztes Oniumsalz** | **Eingesetztes Kaliumsalz oder Phospinsaure oder Methylphosphinate** | **Ionisches Fluid** |
|---|---|---|
| [N(C₂H₅)₃(CH₂-CH₂-CN)] Cl | K F₃P(C₂F₅)₃ | [N(C₂H₅)₃(CH₂-CH₂-CN)] F₃P(C₂F₅)₃ |
| N(C₂H₅)₃(CH₂-CH₂-CN)] Cl | K F₃P(C₄F₉)₃ | N(C₂H₅)₃(CH₂-CH₂-CN)] F₃P(C₄F₉)₃ |
| N(C₂H₅)₃(CH₂-CH₂-CN)]Cl | K OP(O)(C₂F₅)₂ | N(C₂H₅)₃(CH₂-CH₂-CN)] OP(O)(C₂F₅)₂ |
| | HOP(O)(C₂F₅)₂ | |
| | CH₃OP(O)(C₂F₅)₂ | |
| N(C₂H₅)₃(CH₂-CH₂-CN)]Cl | K OP(O)(C₄F₉)₂ | N(C₂H₅)₃(CH₂-CH₂-CN)] OP(O)(C₄F₉)₂ |
| | HOP(O)(C₄F₉)₂ | |
| | CH₃OP(O)(C₄F₉)₂ | |
| [N(CH₃)₂(C₂H₅)(CH₂-CH₂-CN)] Cl | K F₃P(C₂F₅)₃ | (N(CH₃)₂(C₂H₅)(CH₂-CH₂-CN)] F₃P(C₂F₅)₃ |
| [P(C₄H₉)₃(CH₂-CH₂-CN)] Cl | K F₃P(C₂F₅)₃ | [P(C₄H₉)₃(CH₂-CH₂-CN)] F₃P(C₂F₅)₃ |
| 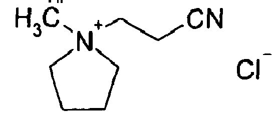 | K F₃P(C₂F₅)₃ | 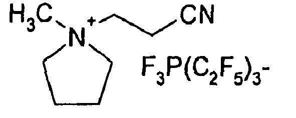 |
| 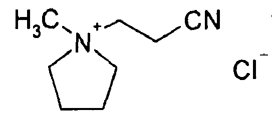 | K F₃P(C₄F₉)₃ | 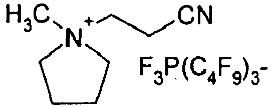 |
| 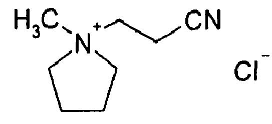 | K OP(O)(C₂F₅)₂ | 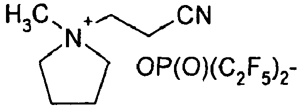 |
| | HOP(O)(C₂F₅)₂ | |
| | CH₃OP(O)(C₂F₅)₂ | |
| 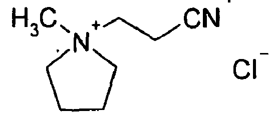 | K OP(O)(C₄F₉)₂ | 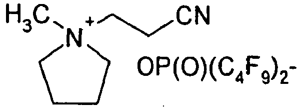 |
| | HOP(O)(C₄F₉)₂ | |
| | CH₃OP(O)(C₄F₉)₂ | |
| 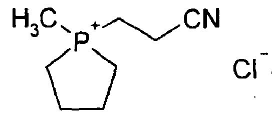 | K F₃P(C₂F₅)₃ | 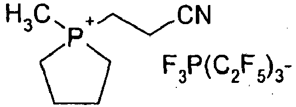 |
| 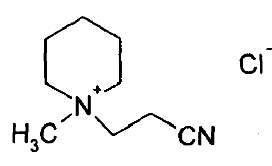 | K F₃P(C₂F₅)₃ | 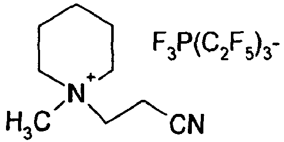 |
| 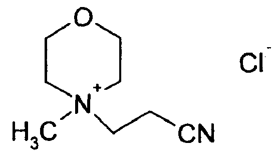 | K F₃P(C₂F₅)₃ | 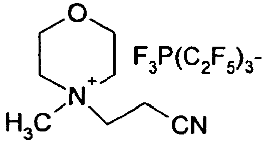 |
| 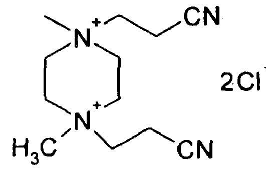 | K F₃P(C₂F₅)₃ | 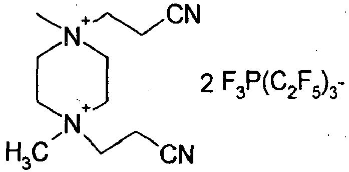 |
| 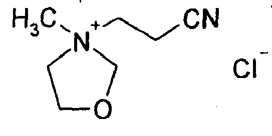 | KF₃P(C₂F₅)₃ | 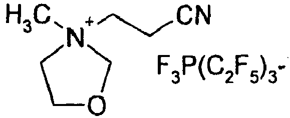 |

### Herstellung der Acetat-, Triflat, Imid- und Methid-basierten reaktiven ionischen Flüssigkeiten

### Beispiel 5: Herstellung der Imid- und Methid-basierten Ionischen Flüssigkeiten

### Allgemeine Vorschrift

In einem 1 Liter- Kolben mit Magnetfisch werden 1 mol des entsprechenden Onium-Chlorids oder Onium-Bromids (aus den vorher beschriebenen Beispielen) in 200 - 500 ml VE-Wasser gelöst oder teilweise suspendiert und anschließend langsam mit 1 Mol des entsprechenden Lithiumsalzes oder Kaliumsalzes oder mit 1 Mol Bis(trifluoromethylsulfonyl)imid (N-H Säure) oder Tris(trifluoromethylsulfonyl)methid (C-H Säure) versetzt. Es bilden sich unmittelbar 2 Phasen.

Diese zwei Phasen werden noch für 1 bis 10 Stunden bei Raumtemperatur nachgerührt. Danach wird die Emulsion 3 mal mit je 50 ml Dichlormethan ausgeschüttelt. Die organische Phase wird dann noch 5 mal mit je 100 ml VE-Wasser Chlorid frei gewaschen (Nachweis: 1 molare Silbernitratlösung).

Die organische Lösung wird noch mit 10 g Al₂O₃ und 1,4 g Aktivkohle versetzt und nach
ca. 1 h wieder filtriert und am Rotationsverdampfer bei einem Wasserbad von ca. 80 °C eingeengt.

**Tab. 7**

| **Eingesetztes Oniumsalz** | **Eingesetztes Lithiumsalz oder Bis(trifluoromethylsulfonyl)imid oder Tris(trifluoromethylsulfonyl)-methid** | **Ionische Flüssigkeit** |
|---|---|---|
| [N(C₂H₅)₃(CH₂-Cl | Li N(SO₂CF₃)₂ | [N(C₂H₅)₃(CH2-CH=CH₂)] N(SO₂CF₃)₂ |
| | HN(SO₂CF₃)₂ | |
| [N(C₂H₅)₃(CH₂-CH=CH₂)] Cl | Li C(SO₂CF₃)₃ | [N(C₂H₅)₃(CH2-CH=CH₂)] |
| | HN(SO₂CF3)₂ | |
| [N(CH₃)(C₂H₅)₃(CH₂-CH=CH₂)] Cl | Li N(SO₂CF₃)₂ | [N(CH3)(C₂H₅)₃(CH2-CH=CH₂)] N(SO₂CF₃)₂ |
| | HN(SO₂CF₃)₂ | |
| [P(C₄H₉)₃(CH₂-CH=CH₂)] Cl | Li N(SO₂CF₃)₂ | [P(C₄H₉)₃(CH₂-CH=CH₂)] N(SO₂CF₃)₂ |
| | HN(SO₂CF3)₂ | |
| 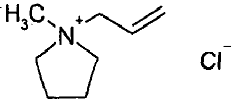 | Li N(SO₂CF₃)₂ | 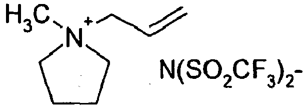 |
| | HN(SO₂CF₃)₂ | |
| 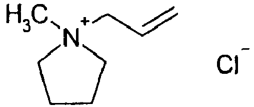 | Li C(SO₂CF₃)₃ | 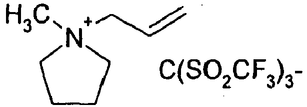 |
| | HC(SO₂CF₃)₃ | |
| 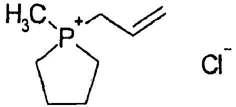 | Li N(SO₂CF₃)₂ | 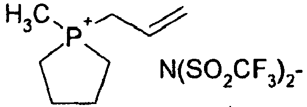 |
| | HN(SO₂CF₃)₂ | |
| 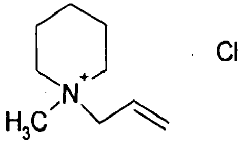 | Li N(SO₂CF₃)₂ | 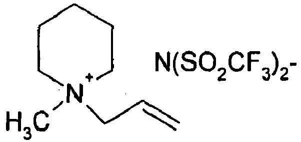 |
| | HN(SO₂CF₃)₂ | |
| 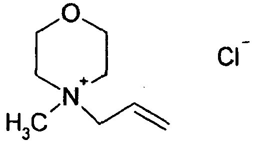 | Li N(SO₂CF₃)₂ | 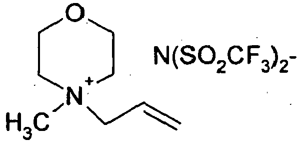 |
| | HN(SO₂CF₃)₂ | |
| 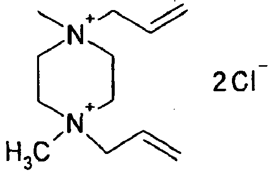 | Li N(SO₂CF₃)₂ | 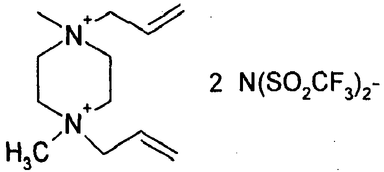 |
| | HN(SO₂CF₃)₂ | |
| 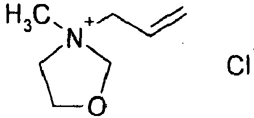 | Li N(SO₂CF₃)₂ | 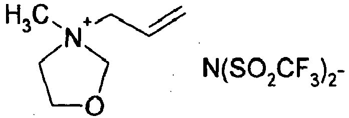 |
| | HN(SO₂CF₃)₂ | |

**Tab.8**

| **Eingesetztes Oniumsalz** | **Eingesetztes Lithiumsalz oder Bis(trifluoromethyl sul-fonyl)imid oder Tris(trifluoromethylsul-fonyl)methid** | **Ionische Flüssigkeit** |
|---|---|---|
| N(C₂H₅)₃(CH₂-C(O)-O-C₂H₅)] Br | Li N(SO₂CF₃)₂ | [N(C₂H₅)₃(CH₂-C(O)-O-C₂H₅)] N(SO₂CF₃)₂ |
| | HN(SO₂CF3)₂ | |
| [N(C₂H₅)₃(CH₂-C(O)-O-C₂H₅)] Cl | Li OSO₂CF₃ | [N(C₂H₅)₃(CH₂-C(O)-O-C₂H₅)] OSO₂CF₃ |
| [N(C₂H₅)₃(CH₂-C(O)-O-C₂H₅)] Cl | Li OCOCF₃ | [N(C₂H₅)₃(CH₂-C(O)-O-C₂H₅)] OCOCF₃ |
| [N(C₂H₅)₃(CH₂-C(O)-O-C₂H₅)] Br | Li C(SO₂CF₃)₃ | [N(C₂H₅)₃(CH₂-C(O)-O-C₂H₅)] C(SO₂CF₃)₃ |
| | HC(SO₂CF₃)₃ | |
| [N(CH₃)₂(C₂H₅) (CH₂-CH₂-C(O)-O-CH₃)] Br | Li N(SO₂CF₃)₂ | [N(CH₃)₂(C₂H₅) (CH₂-CH₂-C(O)-O-CH₃)] N(SO₂CF₃)₂ |
| | HN(SO₂CF3)₂ | |
| [N(CH₃)₂(C₂H₅) (CH₂-C(O)-CH₂-C(O)-O-CH₃)] Br | Li N(SO₂CF₃)₂ | [N(CH₃)₂(C₂H₅) (CH₂-C(O)-CH₂-C(O)-O-CH₃)] N(SO₂CF₃)₂ |
| | HN(SO₂CF₃)₂ | |
| [N(CH₃)₂(C₂H₅) (CH₂-O-C(O)-O-CH₃)] Br | Li N(SO₂CF₃)₂ | [N(CH₃)₂(C₂H₅) (CH₂-O-C(O)-O-CH₃)] N(SO₂CF₃)₂ |
| | HN(SO₂CF3)₂ | |
| [P(C₄H₉)₃(CH₂-C(O)-O-C₂H₅)] Br | Li N(SO₂CF₃)₂ | [P(C₄H₉)₃(CH₂-C(O)-O-C₂H₅)] N(SO₂CF₃)₂ |
| | HN(SO₂CF3)₂ | |
| 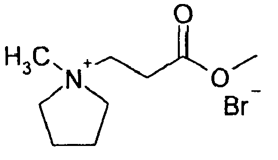 | Li N(SO₂CF₃)₂ | 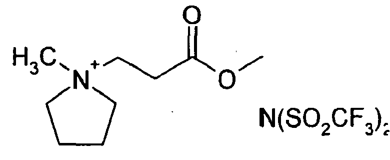 |
| | HN(SO₂CF₃)₂ | |
| 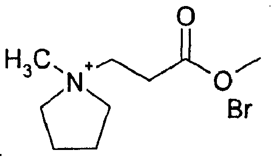 | Li C(SO₂CF₃)₃ | 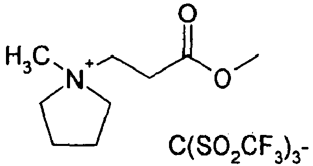 |
| | HC(SO₂CF₃)₃ | |
| 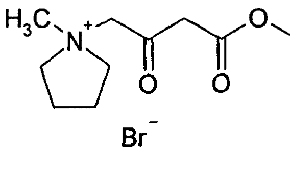 | Li N(SO₂CF₃)₂ | 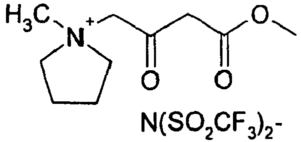 |
| | HN(SO₂CF₃)₂ | |
| 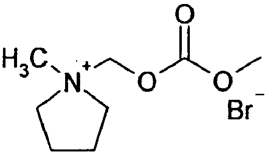 | Li N(SO₂CF₃)₂ | 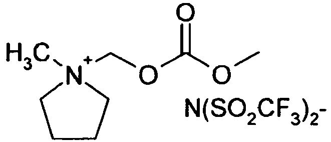 |
| | HN(SO₂CF₃)₂ | |
| 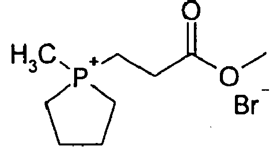 | Li N(SO₂CF₃)₂ | 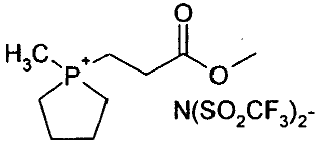 |
| | HN(SO₂CF₃)₂ | |
| 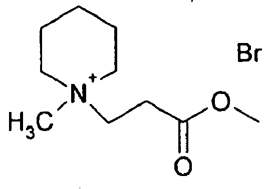 | Li N(SO₂CF₃)₂ | 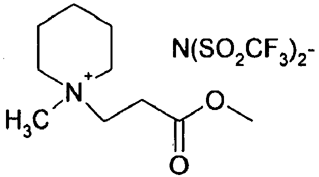 |
| | HN(SO₂CF₃)₂ | |
| 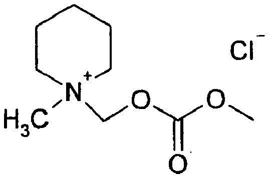 | Li N(SO₂CF₃)₂ | 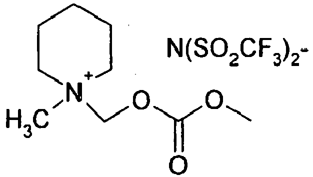 |
| | HN(SO₂CF₃)₂ | |
| 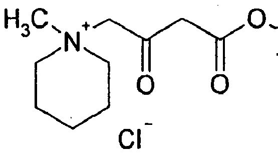 | Li N(SO₂CF₃)₂ | 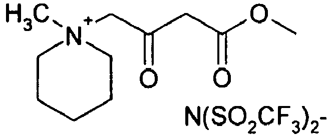 |
| | HN(SO₂CF₃)₂ | |
| 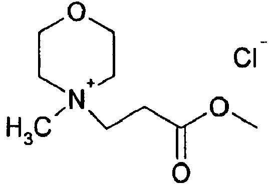 | Li N(SO₂CF₃)₂ | 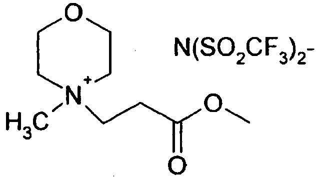 |
| | HN(SO₂CF₃)₂ | |
| 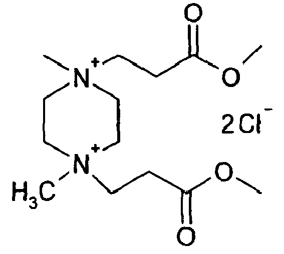 | Li N(SO₂CF₃)₂ | 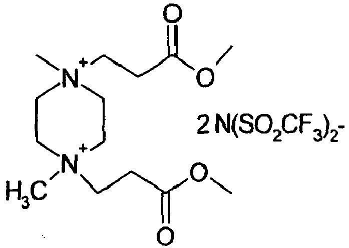 |
| | HN(SO₂CF₃)₂ | |
| 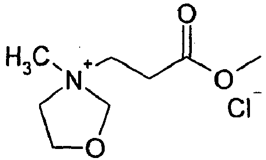 | Li N(SO₂CF₃)₂ | 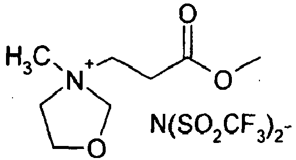 |
| | HN(SO₂CF₃)₂ | |

**Tab. 9**

| **Eingesetztes Oniumsalz** | **Eingesetztes Lithiumsalz oder Bis(trifluoromethyl-sulfonyl)imid oder Tris(trifluoromethyl-sulfonyl)methid** | **Ionische Flüssigkeit** |
|---|---|---|
| [N(C₂H₅)₃(CH₂-CH₂-CN)] Cl | Li N(SO₂CF₃)₂ | [N(C₂H₅)₃(CH₂-CH₂-CN)] N(SO₂CF₃)₂ |
| | HN(SO₂CF3)₂ | |
| N(C₂H₅)₃(CH₂-CH₂-CN)] Cl | Li C(SO₂CF₃)₃ | N(C₂H₅)₃(CH₂-CH₂-CN)] C(SO₂CF₃)₃ |
| | HC(SO₂CF₃)₃ | |
| [N(CH₃)₂(C₂H₅) (CH₂-CH₂-CN)] Cl | Li N(SO₂CF₃)₂ | [N(CH₃)₂(C₂H₅) (CH₂-CH₂-CN)] N(SO₂CF₃)₂ |
| | HN(SO₂CF3)₂ | |
| [P(C₄H₉)₃(CH₂-CH₂-CN)] Cl | Li N(SO₂CF₃)₂ | [P(C₄H₉)₃(CH₂-CH₂-CN)] N(SO₂CF₃)₂ |
| | HN(SO₂CF3)₂ | |
| 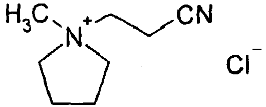 | Li N(SO₂CF₃)₂ | 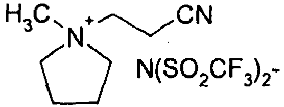 |
| | HN(SO₂CF₃)₂ | |
| 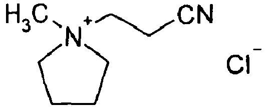 | Li C(SO₂CF₃)₃ | 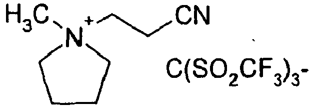 |
| | HC(SO₂CF₃)₃ | |
| 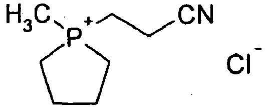 | Li N(SO₂CF₃)₂ | 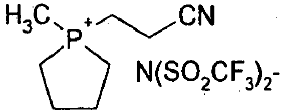 |
| | HN(SO₂CF₃)₂ | |
| 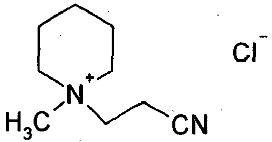 | Li N(SO₂CF₃)₂ | 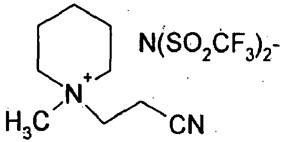 |
| | HN(SO₂CF₃)₂ | |
| 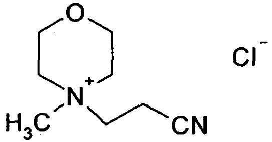 | Li N(SO₂CF₃)₂ | 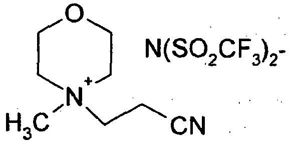 |
| | HN(SO₂CF₃)₂ | |
| 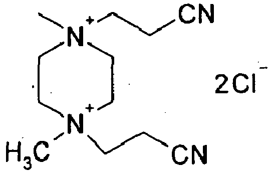 | Li N(SO₂CF₃)₂ | 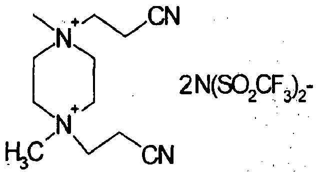 |
| | HN(SO₂CF₃)₂ | |
| 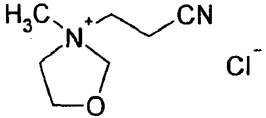 | Li N(SO₂CF₃)₂ | 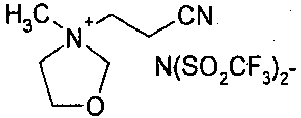 |
| | HN(SO₂CF₃)₂ | |

### Herstellung der Trifluoracetat- oder Triflat-basierten Ionischen Flüssigkeiten

### Beispiel 6.1: Herstellung der Triflat-basierten ionischen Flüssigkeiten mittels Triflic-Säure

### Allgemeine Vorschrift

In einem Kolben mit Magnetfisch werden 1 mol des entsprechenden Onium-Chlorids (aus den vorher beschriebenen Beispielen) in 200 - 500 ml VE-Wasser gelöst und anschließend langsam mit 1 Mol der Triflic-Säure (= Trifluormethansulfonsäure) versetzt.

Die Reaktionsmischung wird noch für 1 h bei Raumtemperatur nachgerührt und Wasser zusammen mit entstehender HCl abdestilliert. Um vollständige Abtrennung von HCl zu erreichen, kann man die mehrmalige azeotropische Destillation mit Dioxan und Wasser benutzen (bis zum negativen Test mit Silbernitratlösung). Nach der Trocknung im Vakuum bei 80° C - 90° C erhält man die entschpechenden Triflate in nahe zu quantitativer Ausbeute.

### Beispiel 6.2: Herstellung der Trifluoracetat- oder Triflat-basierten ionischen Flüssigkeiten über die entsprechenden Kalium- oder Lithiumsalze

### Allgemeine Vorschrift

In einem Kolben mit Magnetfisch werden 1 mol des entsprechenden Onium-Chlorids (aus den vorher beschriebenen Beispielen) in *iso-*Propanol (oder Methanol oder Acetonitril) gelöst und anschließend langsam mit 1 Mol der entsprechenden Kalium- (oder Lithium-) Trifluoroacetate oder -Triflate versetzt.

Die Reaktionsmischung wird noch für 1 h bei Raumtemperatur nachgerührt und entstehendes KCl (oder LiCl) abfiltriert. Nach der Entfernung von *iso-*Propanol (oder Methanol oder Acetonitril) im Vakuum mit Hilfe von Rotationsverdampfer erhält man die Trifluoroacetate oder Triflate in nahe zu quantitativer Ausbeute.

### Beispiel 6.3: Herstellung der Trifluoroacetat- und Triflat-basierten ionischen Flüssigkeiten über die entsprechenden Methyl-Triflate, Trimethylsilyl-Triflate, Triflic-Anhydride oder Trifluoroacetat-Anhydride

In einem Kolben mit Magnetfisch werden 1 mol des entsprechenden Onium-Chlorids (oder Bromids) (aus den vorher beschriebenen Beispielen) mit 1 bis 1,1 Mol der entsprechenden Methyl-Triflate oder Trimethylsilyl-Triflate oder Triflic-Anhydride oder Trifluoroacetat-Anhydride versetzt.

Die Reaktionsmischung wird für 1 bis 48 h bei Raumtemperatur oder bei Heizung bis 100° C nachgerührt und entstehendes CH₃Cl (oder CH₃Br, oder (CH₃)₃SiCl oder CF₃SO₂Cl oder CF₃COCl oder CF₃COBr) und Überschuss von Methyl- oder Trimethylsilyl- Triflate oder -Anhydride im Vakuum entfernt. Trifluoroacetate oder Triflate bilden sich in nahe zu quantitativer Ausbeute.

**Tab. 10**

| **Eingesetztes Oniumsalz** | **Eingesetztes Lithium- oder Kalium- salz oder Methyltriflate oder Anhydride** | **Ionische Flüssigkeiten** |
|---|---|---|
| [N(C₂H₅)₃(CH₂-CH=CH₂)] Cl | K OSO₂CF₃, HOSO₂CF₃, CH₃OSO₂CF₃, (CH₃)₃SiOSO₂CF₃, | [N(C₂H₅)₃(CH₂-CH=CH₂)] |
| [N(C₂H₅)₃(CH2-CH=CH₂)] Cl | Li OCOCF₃, (CF₃CO)₂O | (N(C₂H₅)₃(CH2-CH=CH₂)] |
| 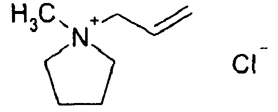 | K OSO₂CF₃, HOSO₂CF₃, CH₃OSO₂CF₃, (CH₃)₃SiOSO₂CF₃, | 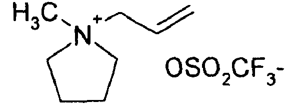 |
| 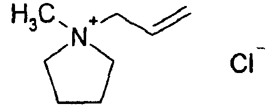 | Li OCOCF₃, (CF₃CO)₂O | 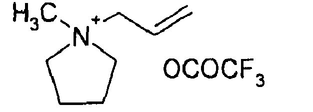 |
| | | |
| [N(C₂H₅)₃(CH₂-C(O)-O-C₂H₅)]Br | K OSO₂CF₃, HOSO₂CF₃, CH₃OSO₂CF₃, (CH₃)₃S)OSO₂CF₃ | [N(C₂H₅)₃(CH₂-C(O)-O-C₂H₅)] OSO₂CF₃ |
| [N(C₂H₅)₃(CH₂-C(O)-O-C₂H₅)] Br | K OCOCF₃, (CF₃CO)₂O | [N(C₂H₅)₃(CH₂-C(O)-O-C₂H₅)] OCOCF₃ |
| 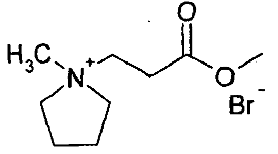 | K OSO₂CF₃, HOSO₂CF₃, CH₃OSO₂CF₃, (CH₃)₃SiOSO₂CF₃ | 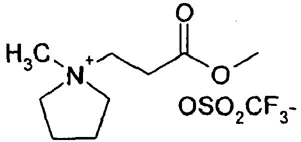 |
| 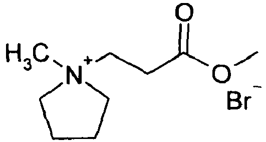 | KOCOCF₃, (CF₃CO)₂O | 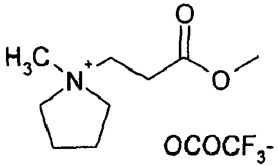 |
| 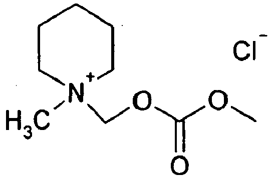 | K OSO₂CF₃, HOSO₂CF₃, CH₃OSO₂CF₃, (CH₃)₃SiOSO₂CF₃ | 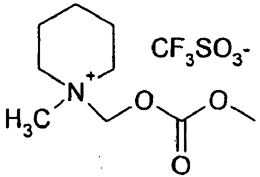 |
| 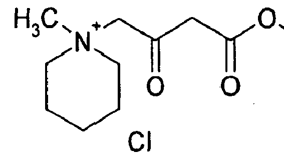 | K OCOCF₃, (CF₃CO)₂O | 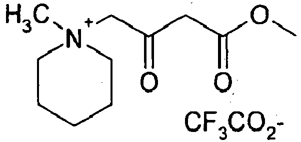 |
| 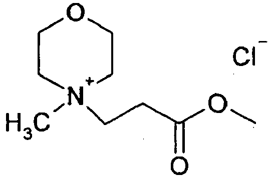 | K OSO₂CF₃, HOSO₂CF₃, CH₃OSO₂CF₃, (CH₃)₃SiOSO₂CF₃ | 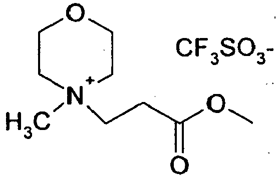 |
| 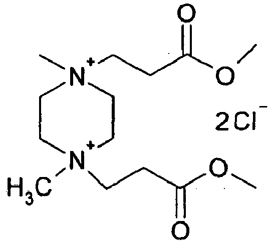 | K OSO₂CF₃, HOSO₂CF₃, CH₃OSO₂CF₃, (CH₃)₃SiOSO₂CF₃ | 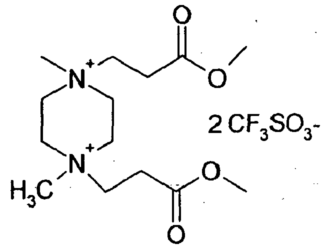 |
| 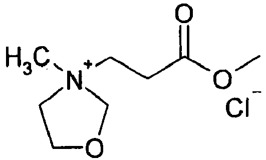 | K OSO₂CF₃, HOSO₂CF₃, CH₃OSO₂CF₃, (CH₃)₃SiOSO₂CF₃ | 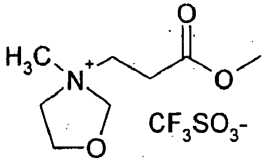 |

**Tab. 11**

| **Eingesetztes Oniumsalz** | **Eingesetztes Lithium- oder Kaliumsalz oder Methyltriflate oder Anhydride** | **Ionische Flüssigkeiten** |
|---|---|---|
| N(C₂H₅)₃(CH₂-CH₂-CN)]Cl | K OSO₂CF₃, HOSO₂CF₃, CH₃OSO₂CF₃, (CH₃)₃SiOSO₂CF₃ | N(C₂H₅)₃(CH₂-CH₂-CN)] OSO₂CF₃ |
| N(C₂H₅)₃(CH₂-CH₂-CN)]Cl | K OCOCF₃, (CF₃CO)₂O | N(C₂H₅)₃(CH₂-CH₂-CN)] OCOCF₃ |
| 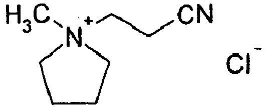 | K OSO₂CF₃, HOSO₂CF₃, CH₃OSO₂CF₃, (CH₃)₃SiOSO₂CF₃ | 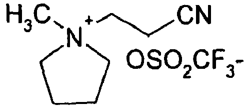 |
| 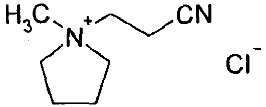 | K OCOCF₃, (CF₃CO)₂O | 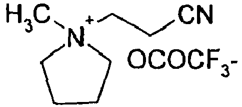 |
| 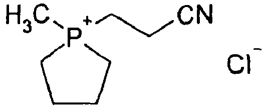 | K OSO₂CF₃, CH₃OSO₂CF₃, (CH₃)₃SiOSO₂CF₃ | 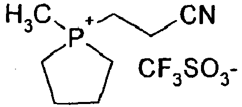 |
| 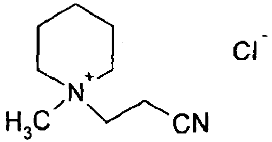 | K OSO₂CF₃, HOSO₂CF₃, CH₃OSO₂CF₃, (CH₃)₃SiOSO₂CF₃ | 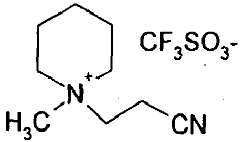 |
| 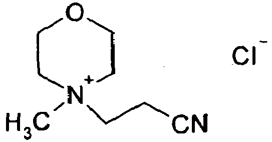 | K OSO₂CF₃, HOSO₂CF₃, CH₃OSO₂CF₃, (CH₃)₃SiOSO₂CF₃ | 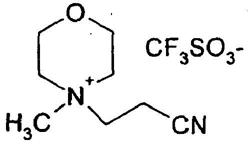 |
| 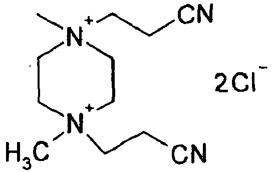 | K OSO₂CF₃, HOSO₂CF₃, CH₃OSO₂CF₃, (CH₃)₃SiOSO₂CF₃ | 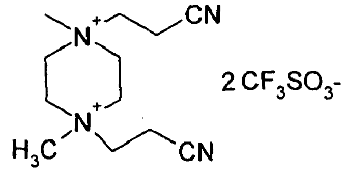 |
| 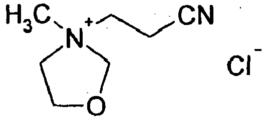 | K OSO₂CF₃, HOSO₂CF₃, CH₃OSO₂CF₃, (CH₃)₃SiOSO₂CF₃ | 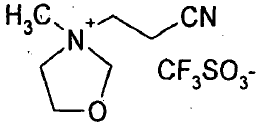 |

### Beispiel 7: Herstellung der Borat-, Phosphat- und Aluminat-basierten ionischen Flüssigkeiten

### Allgemeine Vorschrift

In einem 3 Liter- Kolben mit Magnetfisch werden 1 mol des entsprechenden Onium-Chlorids oder Onium-Bromids (aus den vorher beschriebenen Beispielen) in 2 I Dichlormethan gelöst und anschließend langsam mit 1 Mol des entsprechenden Kalium- oder Lithiumsalzes versetzt. Nach Zugabe wird die Reaktionsmischung 5 Tage gerührt. Danach wird der Ansatz mit 500 ml VE-Wasser versetzt. Es bilden sich 2 Phasen. Die organische Phase wird abgetrennt, die wässrige Phase mit 300 ml Dichlormethan gewaschen und alle organischen Phasen vereinigt. Die organische Phase wird noch 2 mal mit ja 250 ml VE-Wasser gewaschen und danach auf 1/3 eingeengt und anschließend in 1 L n-Heptan eingerührt.

Es bilden sich zwei Phasen. Die n-Heptan-Phase mit der ionischen Flüssigkeit wird wurde über Nacht abgetrennt und am Rotationsverdampfer bei einem Wasserbad von 60 bis 70 °C eingeengt.

**Tab. 12**

| **Eingesetztes Oniumsalz** | **Eingesetztes Kalium bzw. Lithiumsalz** | **Ionische Flüssigkeit** |
|---|---|---|
| [N(C₂H₅)₃(CH2-CH=CH₂)]Cl | 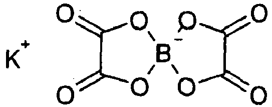 | [N(C₂H₅)₃(CH₂-CH=CH₂)] BOB |
| | Kalium Bis Oxalato Borat (KBOB) | |
| [N(C₂H₅)₃(CH2-CH=CH₂)]Cl | 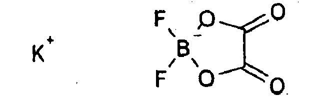 | [N(C₂H₅)₃(CH₂-CH=CH₂)] DFOB |
| | Kalium Di-Fluoro Oxalato Borat (KDFOB) | |
| [N(C₂H₅)₃(CH2-CH=CH₂)]Cl | 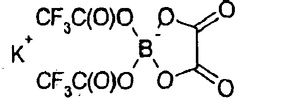 | [N(C₂H₅)₃(CH₂-CH=CH₂)]D(Ac)OB |
| | Kalium Di-Trifluoracetato Oxalato Borat (KD(Ac)OB) | |
| [N(C₂H₅)₃(CH2-CH=CH₂)]Cl | 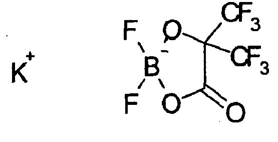 | [N(C₂H₅)₃(CH₂-CH=CH₂)] DFGB |
| | **Kalium Di-Fluor (Di-Trifluomethyl) Glykonato Borat (KDFGB)** | |
| [N(C₂H₅)₃(CH2-CH=CH₂)]Cl | 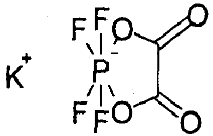 | [N(C₂H₅)₃(CH₂-CH=CH₂)] TFOP |
| | **Kaliun Tetra-Fluor Oxalato Phosphat (KTFOP)** | |
| [N(C₂H₅)₃(CH2-CH=CH₂)]Cl | 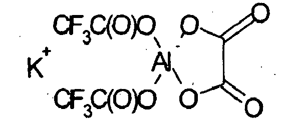 | [N(C₂H₅)₃(CH₂-CH=CH₂)] D(Ac)OAl |
| | Kalium Di-(Trifluoracetato) Oxalato Aluminat (KD(Ac)OAl) | |
| | | |
| [N(C₂H₅)₃(CH2-CH=CH₂)]Cl | 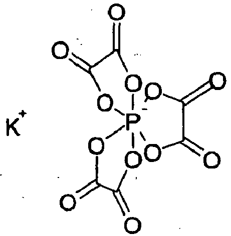 | [N(C₂H₅)₃(CH₂-CH=CH₂)] TOP |
| | Kalium Tris Oxalato Phosphat (KTOP) | |
| [N(C₂H₅)₃(CH2-CH=CH₂)]Cl | 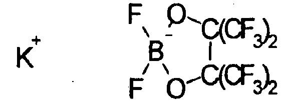 | [N(C₂H₅)₃(CH₂-CH=CH₂)] DFPB |
| | **Kalium Di-Fluoro Perfluor-pinakolato Borat (KDFPB)** | |
| [N(CH₃)(C₂H₅)₃(C H₂-CH=CH₂)]Cl | K oder Li BOB | [N(CH₃)(C₂H₅)₃ (CH₂-CH=CH₂)] BOB |
| [N(CH₃)(C₂H₅)₃(C H₂-CH=CH₂)]Cl | K oder Li DFOB | [N(CH₃)(C₂H₅)₃ (CH₂-CH=CH₂)] DFOB |
| [P(C₄H₉)₃(CH₂-CH=CH₂)]Cl | K oder Li BOB | [P(C₄H₉)₃(CH₂-CH=CH₂)] BOB |
| [P(C₄H₉)₃(CH₂-CH=CH₂)]Cl | K oder Li DFOB | [P(C₄H₉)₃(CH₂-CH=CH₂)] DFOB |
| 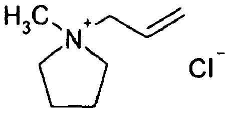 | K oder Li BOB | 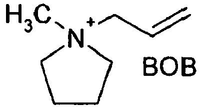 |
| 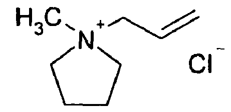 | K oder Li DFOB | 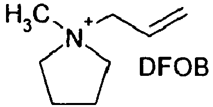 |
| 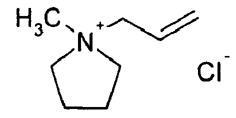 | K oder Li TOP | 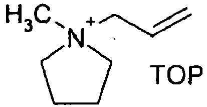 |
| 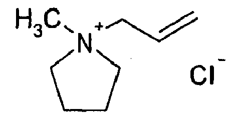 | K oder Li TFOP | 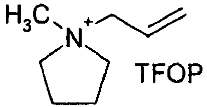 |
| 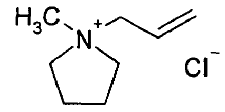 | K oder Li D(Ac)OAl | 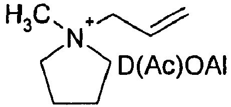 |
| 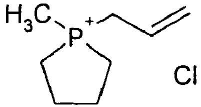 | K oder Li BOB | 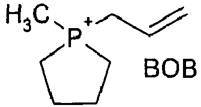 |
| | | |
| 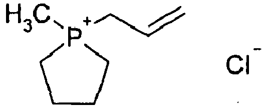 | K oder Li DFOB | 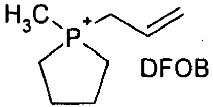 |
| 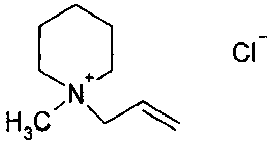 | K oder Li BOB | 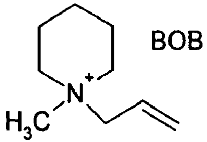 |
| 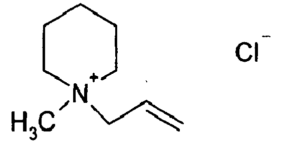 | K oder Li DFOB | 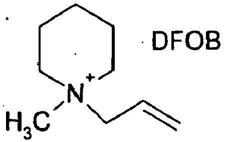 |
| 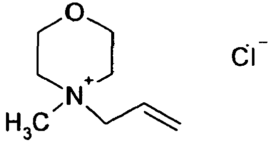 | K oder Li BOB | 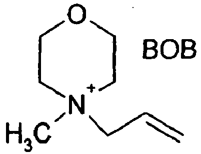 |
| 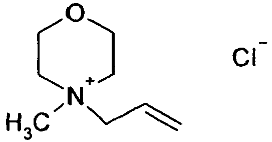 | K oder Li DFOB | 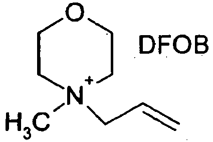 |
| 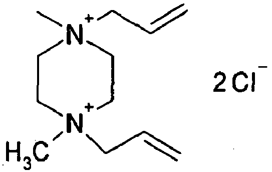 | K oder Li BOB | 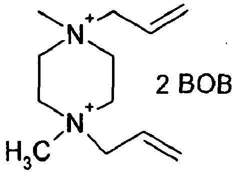 |
| 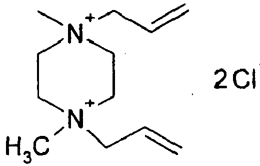 | K oder Li DFOB | 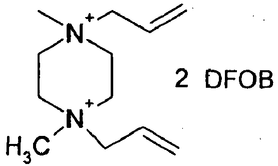 |
| 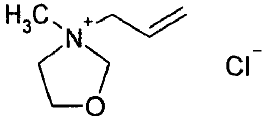 | K oder Li BOB | 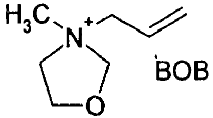 |
| 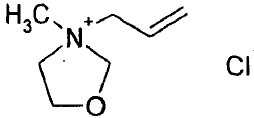 | K oder Li DFOB | 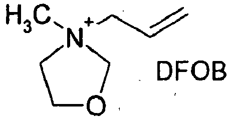 |

**Tab. 13**

| **Eingesetztes Oniumsalz** | **Eingesetztes Kalium-oder Lithium-salz** | **Ionische Flüssigkeit** |
|---|---|---|
| [N(C₂H₅)₃(CH₂-C(O)-O-C₂H₅)]Br | K oder Li BOB | [N(C₂H₅)₃(CH₂-C(O)-O-C₂H₅)] BOB |
| [N(C₂H₅)₃(CH₂-C(O)-O-C₂H₅)]Br | K oder Li DFOB | [N(C₂H₅)₃(CH₂-C(O)-O-C₂H₅)] DFOB |
| [N(C₂H₅)₃(CH₂-C(O)-O-C₂H₅)]Br | K oder Li TOP | [N(C₂H₅)₃(CH₂-C(O)-O-C₂H₅)] TOP |
| [N(C₂H₅)₃(CH₂-C(O)-O-C₂H₅)]Br | K oder Li D(Ac)OAl | [N(C₂H₅)₃(CH₂-C(O)-O-C₂H₅)] D(Ac)Oal |
| [N(CH₃)₂(C₂H₅)(CH₂-CH₂-C(O)-O-CH₃)] Br | K oder Li BOB | [N(CH₃)₂(C₂H₅)(CH₂-CH₂-C(O)-O-CH₃)] BOB |
| [N(CH₃)₂(C₂H₅)(CH₂-CH₂-C(O)-O-CH₃)] Br | K oder Li DFOB | [N(CH₃)₂(C₂H₅)(CH₂-CH₂-C(O)-O-CH₃)] DFOB |
| [N(CH₃)₂(C₂H₅)(CH₂-C(O)-CH₂-C(O)-O-CH₃)] Br | K oder Li BOB | [N(CH₃)₂(C₂H₅)(CH₂-C(O)-CH₂-C(O)-O-CH₃)] BOB |
| [N(CH₃)₂(C₂H₅)(CH₂-C(O)-CH₂-C(O)-O-CH₃)] Br | K oder Li DFOB | [N(CH₃)₂(C₂H₅)(CH₂-C(O)-CH₂-C(O)-O-CH₃)] BDFOB |
| [N(CH₃)₂(C₂H₅)(CH₂-O-C(O)-O-CH₃)]Br | K oder Li BOB | [N(CH₃)₂(C₂H₅)(CH₂-O-C(O)-O-CH₃)] BOB |
| [N(CH₃)₂(C₂H₅)(CH₂-O-C(O)-O-CH₃)] Br | K oder Li DFOB | [N(CH₃)₂(C₂H₅)(CH₂-O-C(O)-O-CH₃)] DFOB |
| [P(C₄H₉)₃(CH₂-C(O)-O-C₂H₅)]Br | K oder Li BOB | [P(C₄H₉)₃(CH₂-C(O)-O-C₂H₅)] BOB |
| [P(C₄H₉)₃(CH₂-C(O)-O-C₂H₅)] Br | K oder Li DFOB | [P(C₄H₉)₃(CH₂-C(O)-O-C₂H₅)]DFOB |
| 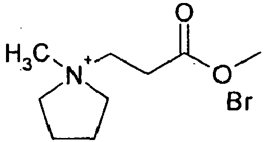 | K oder Li BOB | 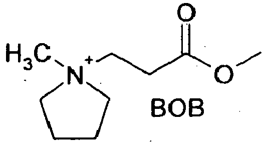 |
| 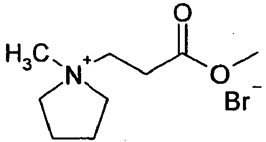 | K oder Li DFOB | 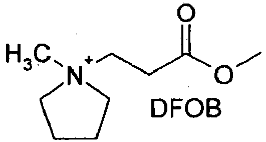 |
| 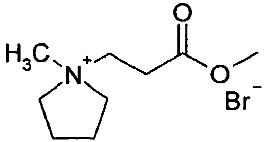 | K oder Li TOP | 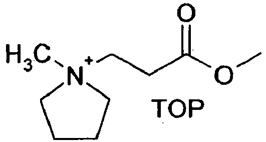 |
| 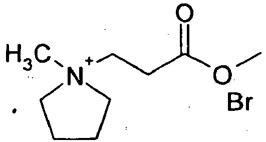 | K oder Li D(Ac)OAl | 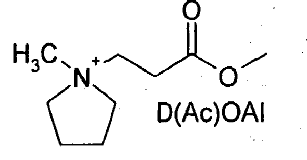 |
| 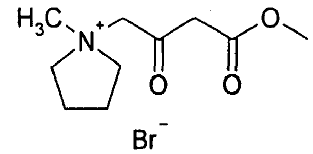 | K oder Li BOB | 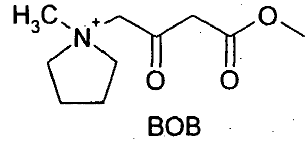 |
| 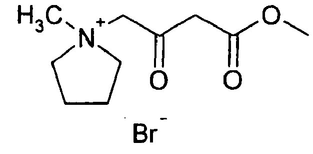 | K oder Li DFOB | 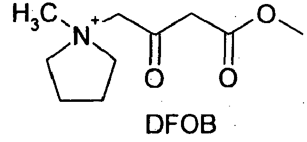 |
| 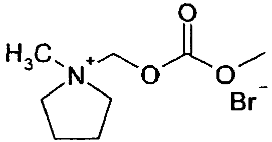 | K oder Li BOB | 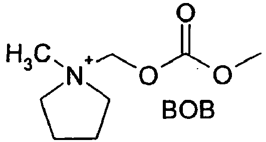 |
| 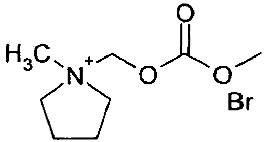 | K oder Li DFOB | 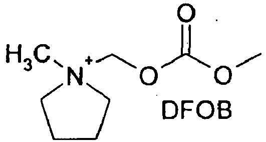 |
| 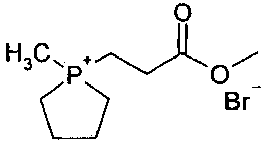 | K oder Li BOB | 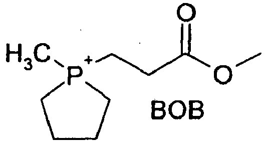 |
| 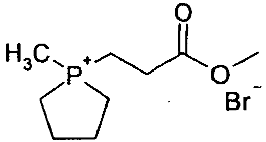 | K oder Li DFOB | 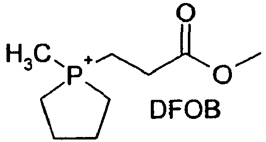 |
| 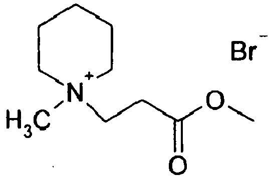 | K oder Li BOB | 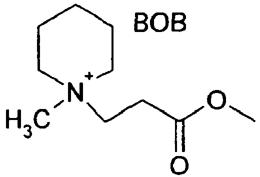 |
| 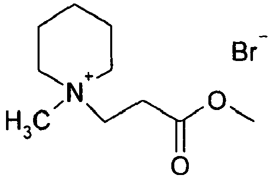 | K oder Li DFOB | 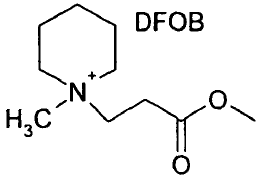 |
| 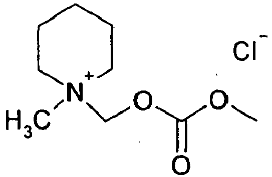 | K oder Li BOB | 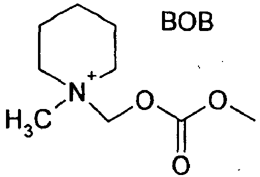 |
| 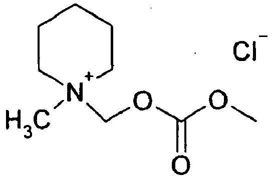 | K oder Li DFOB | 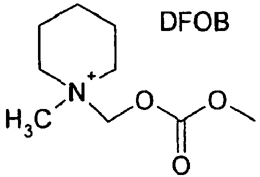 |
| 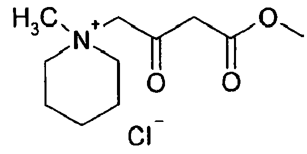 | K oder Li BOB | 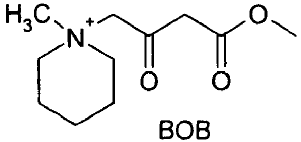 |
| 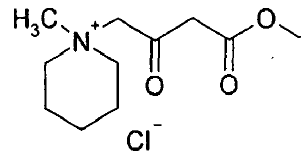 | K oder Li DFOB | 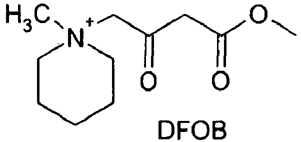 |
| 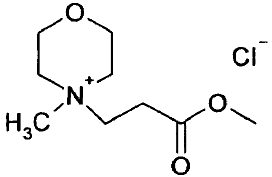 | K oder Li BOB | 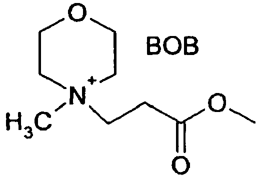 |
| 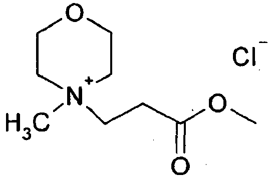 | K oder Li DFOB | 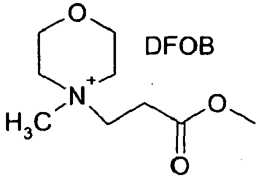 |
| 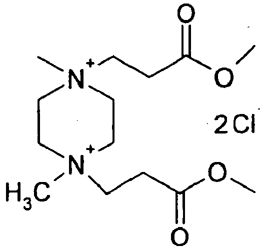 | K oder Li BOB | 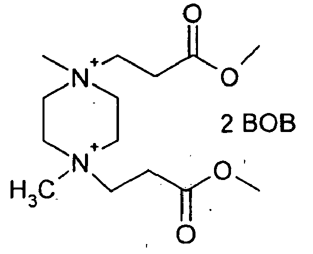 |
| 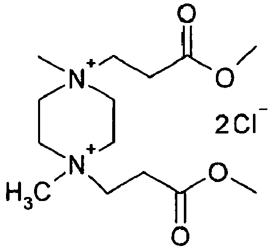 | K oder Li DFOB | 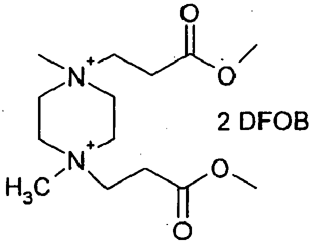 |
| 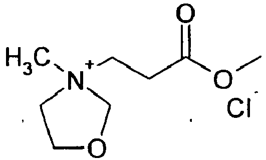 | K oder Li BOB | 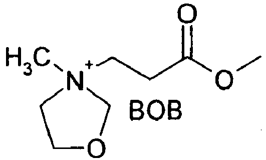 |
| 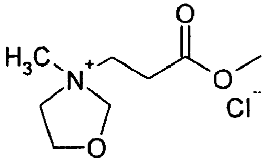 | K oder Li DFOB | 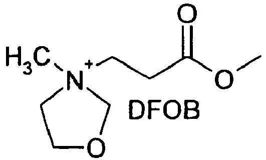 |

**Tab. 14**

| **Eingesetztes Oniumsalz** | **Eingesetztes Lithium- oder Kaliumsalz oder Methyltriflate oder Anhydride** | **Ionische Flüssigkeiten** |
|---|---|---|
| N(C2H5)3(CH2-CH2-CN)] Cl | LiBOB oder KBOB | N(C2H5)3(CH2-CH2-CN)] BON |
| N(C2H5)3(CH2-CH2-CN)] Cl | K oder Li DFOB | N(C2H5)3(CH2-CH2-CN)]DFOB |
| 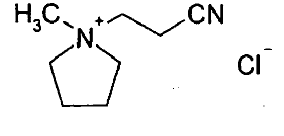 | K oder Li DFOB | 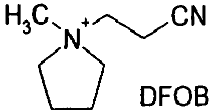 |
| 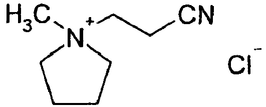 | LiBOB oder KBOB | 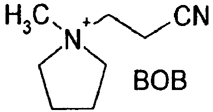 |
| 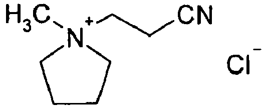 | K oder LiTOP | 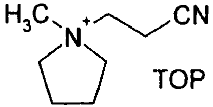 |
| 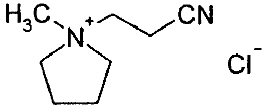 | K oder Li D(Ac)OAl | 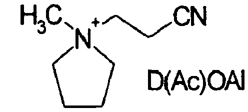 |
| 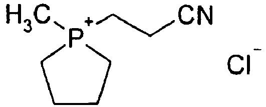 | K oder Li DFOB | 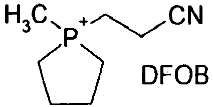 |
| 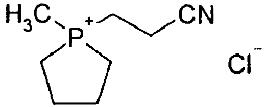 | LiBOB oder KBOB | 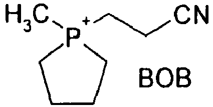 |
| 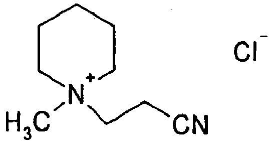 | K oder Li DFOB | 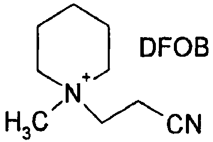 |
| 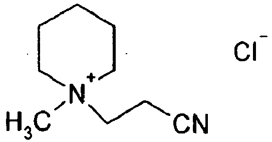 | K oder Li DFOB | 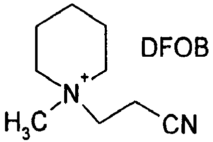 |
| 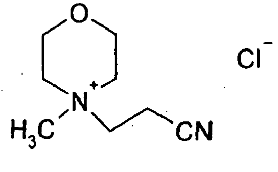 | LiBOB oder KBOB | 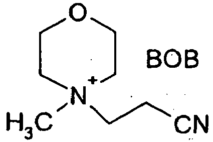 |
| 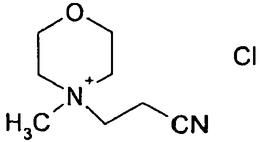 | K oder Li DFOB | 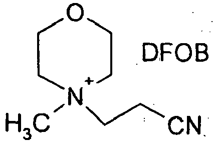 |
| 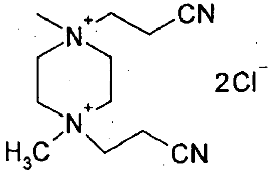 | K oder Li DFOB | 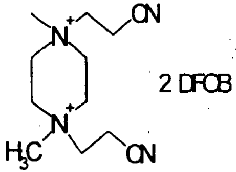 |
| 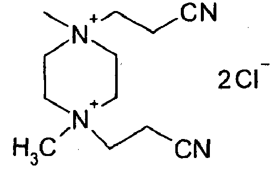 | LiBOB oder KBOB | 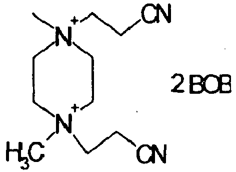 |
| 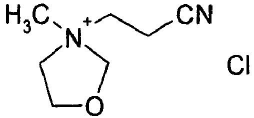 | K oder Li DFOB | 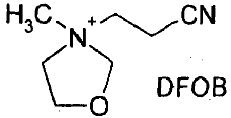 |
| 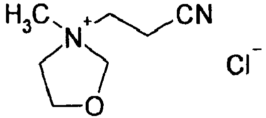 | LiBOB oder KBOB | 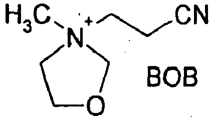 |

### Beispiel 8: Untersuchungen an Graphit

In einer Messzelle mit Graphitanode (SFG 44 mit PVDF-Binder) Lithiumgegenelektrode und Lithiumreferenzelektrode werden jeweils 5 Zyklovoltammogramme hintereinander aufgenommen. Hierzu wird ausgehend vom Ruhepotential das Potential zuerst mit einer Vorschubgeschwindigkeit von 0,1 mV/s auf 0 V gegen Li/Li⁺ erniedrigt und im weiteren Verlauf zurück auf das Ruhepotential gefahren. Als Elektrolyt wird 1 M LiPF₆ in Ethylencarbonat :Diethylencarbonat (Verhältnis 3 : 7) verwendet zu dem jeweils zwischen ca. 2 % einer reaktiven ionischen Flüssigkeit ausgewählt aus Tabelle 7 gegeben wird. Die reaktive Ionische Flüssigkeit bildet bei Potentialen zwischen ca. 2 V und 0,9 V gegen Li/Li⁺ eine passivierende Deckschicht. Ab dem zweiten Zyklus wird eine 95%ige Ausbeute (± 5% Messtoleranz) der Ein- und Auslagerung von Lithiumionen in den Graphit erzielt. Eine Kointerkalation der reaktiven Ionischen Flüssigkeit ausgewählt aus Tabelle 15 in den Graphit kann nicht beobachtet werden.

**Tab. 15**

| Ionische Flüssigkeit | Ausbeute in % | | | | |
|---|---|---|---|---|---|
| | Zyklus 1 | Zyklus 2 | Zyklus 3 | Zyklus 4 | Zyk lus 5 |
| Referenz kein IL Additiv | 80 | 82 | 90 | 93 | 95 |
| 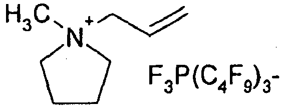 | 75 | 95 | 98 | 99 | 99 |
| 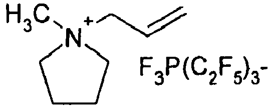 | 76 | 98 | 99 | 99 | 99 |
| 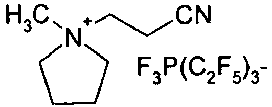 | 72 | 96 | 98 | 98 | 99 |
| 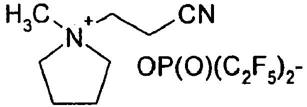 | 70 | 95 | 98 | 99 | 99 |
| 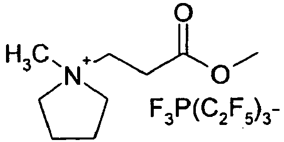 | 77 | 97 | 99 | 99 | 100 |
| 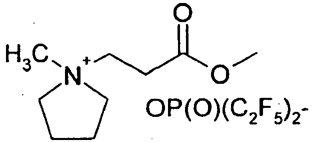 | 72 | 95 | 97 | 98 | 99 |
| 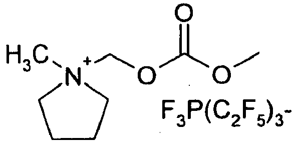 | 78 | 97 | 99 | 99 | 100 |
| 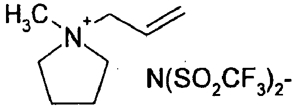 | 75 | 94 | 96 | 99 | 99 |
| 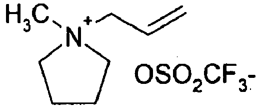 | 72 | 93 | 94 | 97 | 99 |
| 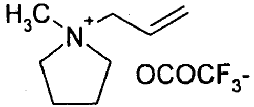 | 74 | 99 | 95 | 99 | 99 |
| 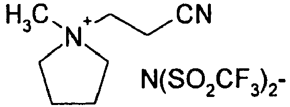 | 74 | 96 | 98 | 98 | 99 |
| 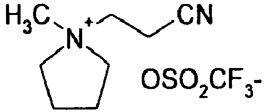 | 72 | 95 | 98 | 99 | 99 |
| 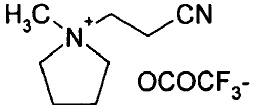 | 72 | 95 | 98 | 99 | 99 |
| 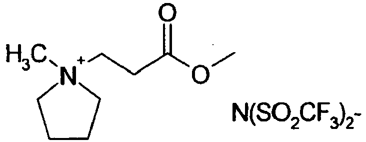 | 77 | 97 | 99 | 99 | 100 |
| 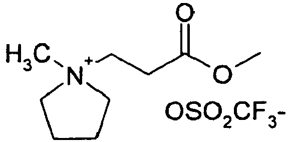 | 72 | 94 | 97 | 98 | 99 |
| 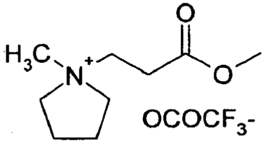 | 72 | 95 | 98 | 98 | 99 |
| 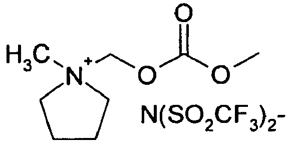 | 80 | 97 | 99 | 99 | 100 |
| 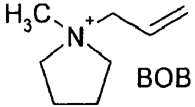 | 75 | 95 | 97 | 99 | 100 |
| 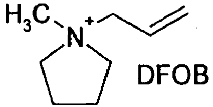 | 72 | 93 | 97 | 98 | 99 |
| 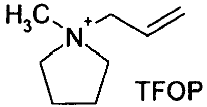 | 74 | 93 | 95 | 99 | 99 |
| 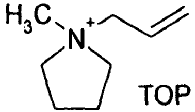 | 74 | 95 | 96 | 99 | 99 |
| 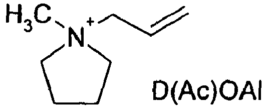 | 72 | 93 | 95 | 98 | 99 |
| 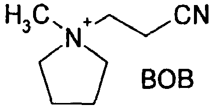 | 74 | 96 | 98 | 98 | 99 |
| 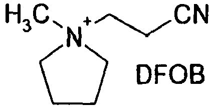 | 72 | 93 | 96 | 98 | 99 |
| 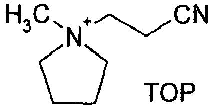 | 72 | 94 | 96 | 98 | 99 |
| | | | | | |
| 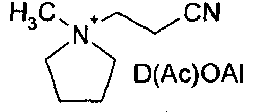 | 72 | 95 | 98 | 99 | 99 |
| 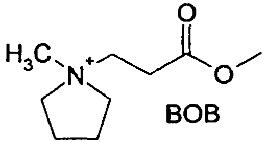 | 77 | 97 | 99 | 100 | 100 |
| 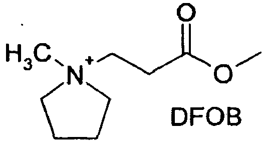 | 72 | 94 | 97 | 98 | 99 |
| 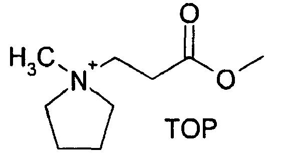 | 72 | 95 | 98 | 98 | 99 |
| 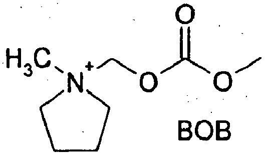 | 80 | 97 | 99 | 100 | 100 |
| 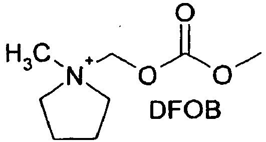 | 80 | 97 | 99 | 99 | 100 |

| | | | | | |
|---|---|---|---|---|---|
| *Referenz = LiPF₆ in EC : DEC (3:7) | | | | | |

### Beispiel 9: Untersuchungen an Graphit

In einer Messzelle mit Graphitanode (SFG 44 mit PVDF-Binder) Lithiumgegenelektrode und Lithiumreferenzelektrode werden jeweils 5 Zyklovoltammogramme hintereinander aufgenommen. Hierzu wird ausgehend vom Ruhepotential das Potential zuerst mit einer Vorschubgeschwindigkeit von 0,1 mV/s auf 0 V gegen Li/Li⁺ erniedrigt und im weiteren Verlauf zurück auf das Ruhepotential gefahren.

Als Elektrolyt wird 1 M LiPF₆ in EC:DEC (3 : 7) verwendet zu dem jeweils zwischen ca. 10% einer reaktiven Ionischen Flüssigkeit ausgewählt aus Tabelle **8** gegeben wird. Die reaktive ionische Flüssigkeit bildet bei Potentialen zwischen ca. 2 V und 0,9 V gegen Li/Li⁺ eine passivierende Deckschicht. Ab dem zweiten Zyklus wird eine über 90%ige Ausbeute der Ein- und Auslagerung von Lithiumionen in den Graphit erzielt.

Eine Kointerkalation der reaktiven ionischen Flüssigkeit aus Tabelle 16 in den Graphit kann nicht beobachtet werden.

**Tab. 16**

| Ionische Flüssigkeit (IL) | Ausbeute in % | | | | |
|---|---|---|---|---|---|
| | Zyklus 1 | Zyklus 2 | Zyklus 3 | Zyklus 4 | Zyklus 5 |
| Referenz* kein IL Additiv | 80 | 82 | 90 | 93 | 95 |
| 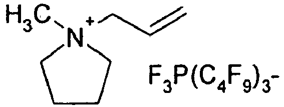 | 70 | 93 | 98 | 99 | 99 |
| 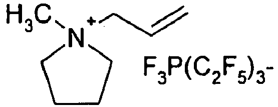 | 72 | 96 | 99 | 99 | 99 |
| 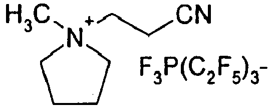 | 69 | 90 | 98 | 98 | 99 |
| 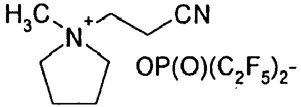 | 69 | 95 | 98 | 99 | 99 |
| 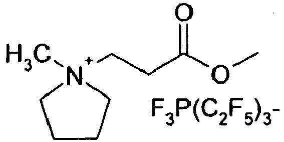 | 72 | 97 | 99 | 99 | 100 |
| 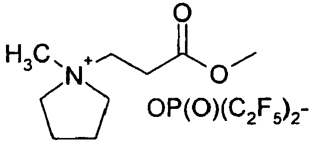 | 70 | 92 | 97 | 98 | 99 |
| 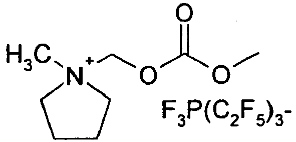 | 75 | 95 | 99 | 99 | 100 |
| 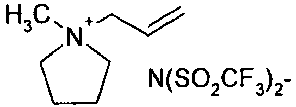 | 70 | 92 | 95 | 98 | 99 |
| 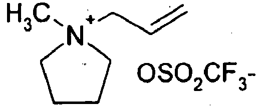 | 69 | 90 | 93 | 97 | 99 |
| 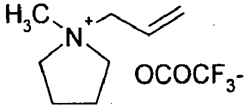 | 72 | 90 | 95 | 99 | 99 |
| 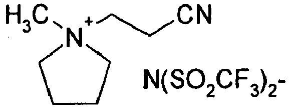 | 70 | 92 | 97 | 98 | 99 |
| 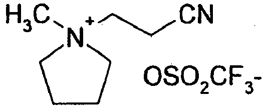 | 72 | 90 | 95 | 99 | 99 |
| 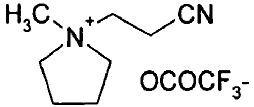 | 72 | 92 | 98 | 99 | 99 |
| 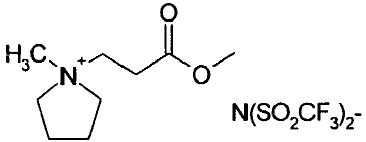 | 73 | 95 | 99 | 99 | 100 |
| 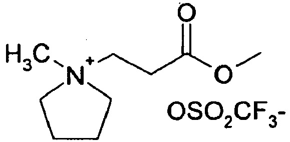 | 70 | 92 | 97 | 98 | 99 |
| 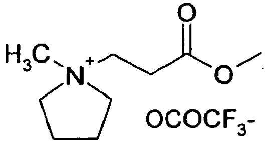 | 73 | 95 | 98 | 98 | 99 |
| 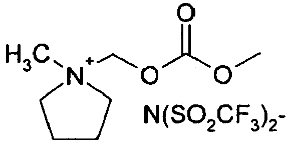 | 75 | 97 | 99 | 99 | 100 |
| 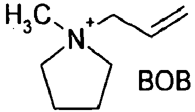 | 70 | 90 | 96 | 98 | 100 |
| 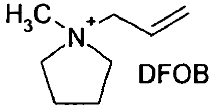 | 69 | 91 | 96 | 97 | 99 |
| 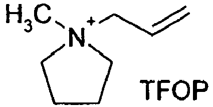 | 71 | 91 | 95 | 98 | 99 |
| | | | | | |
| 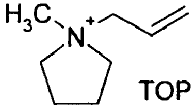 | 72 | 92 | 94 | 99 | 99 |
| 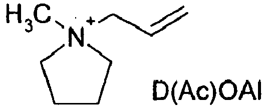 | 70 | 90 | 92 | 97 | 99 |
| 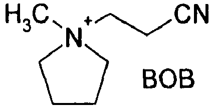 | 71 | 94 | 97 | 98 | 99 |
| 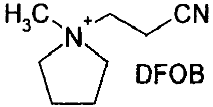 | 70 | 91 | 94 | 97 | 99 |
| 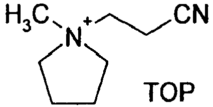 | 69 | 93 | 94 | 98 | 99 |
| 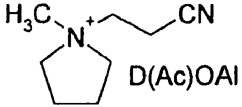 | 69 | 93 | 97 | 98 | 99 |
| 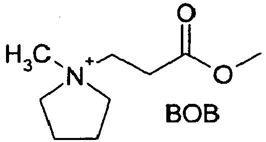 | 75 | 95 | 98 | 198 | 100 |
| 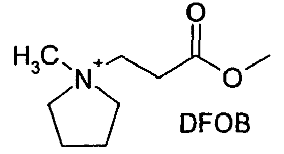 | 71 | 91 | 97 | 98 | 99 |
| 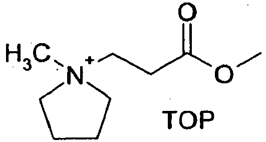 | 70 | 93 | 96 | 98 | 99 |
| | | | | | |
| 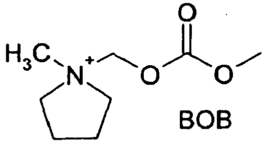 | 78 | 97 | 99 | 100 | 100 |
| 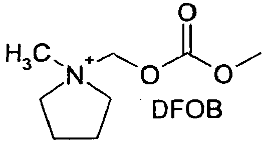 | 78 | 97 | 99 | 99 | 100 |

| | | | | | |
|---|---|---|---|---|---|
| * Referenz = LiPF₆ in EC : DEC (3:7) | | | | | |

### Beispiel 10: Untersuchungen zur Oxidationsstabilität

In einer Messzelle mit Platinarbeitselektrode, Lithiumgegenelektrode und Lithiumreferenzelektrode wurden jeweils 5 Zyklovoltammogramme hintereinander **aufgenommen.** Hierzu wird **ausgehend** vom Ruhepotential **das Potential zuerst mit einer Vorschubgeschwindigkeit** von 10 mV/s auf **6,0 V gegen Li/Li⁺ erhöht und im weiteren Verlauf** zurück auf das

### Ruhepotential gefahren.

Als Elektrolyt wird 1M LiPF₆ in EC:DEC (3 : 7) verwendet zu dem jeweils zwischen ca. 2 % einer reaktiven Ionischen Flüssigkeit ausgewählt aus Tabelle 17 gegeben wird. Das Oxidationspotential wird zu > 5V gegen Li/Li⁺ bestimmt.

In keinem der untersuchten System wird ein Signal gefunden, das unter der Oxidationsstabilität des Referenzelektrolyten liegt.

**Tab. 17**

| | Oxidationsstabilität Eₒₓ |
|---|---|
| Referenz* kein IL Additiv | > 5V |
| 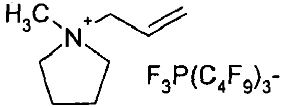 | > 5V |
| 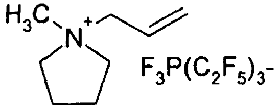 | > 5V |
| 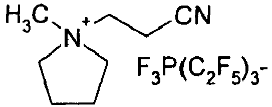 | > 5V |
| 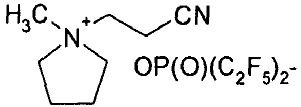 | > 5V |
| 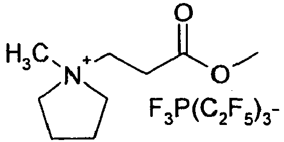 | > 5V |
| 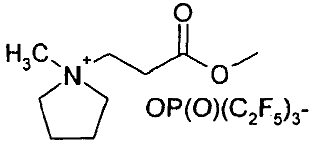 | > 5V |
| 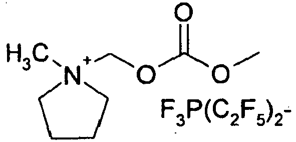 | > 5V |
| 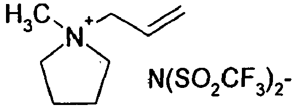 | > 5V |
| 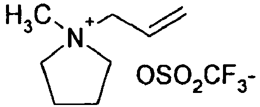 | > 5V |
| 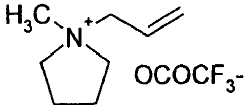 | > 5V |
| 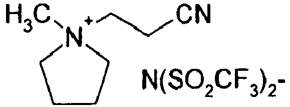 | > 5V |
| 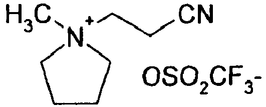 | > 5V |
| 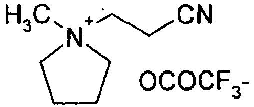 | > 5V |
| 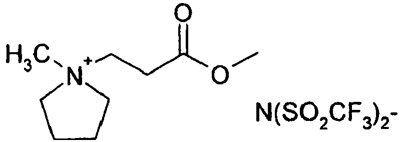 | > 5V |
| 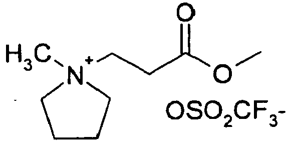 | > 5V |
| 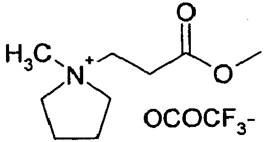 | > 5V |
| 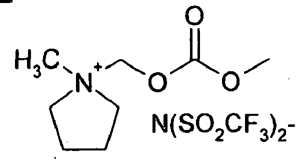 | > 5V |
| 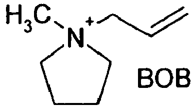 | > 5V |
| 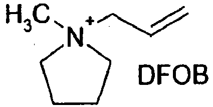 | > 5V |
| 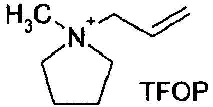 | > 5V |
| 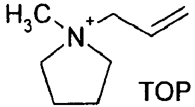 | > 5V |
| 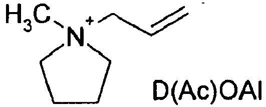 | > 5V |
| 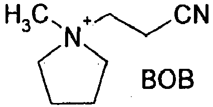 | > 5V |
| 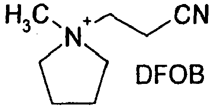 | > 5V |
| 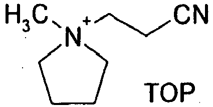 | > 5V |
| 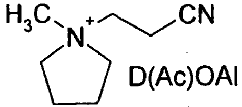 | > 5V |
| 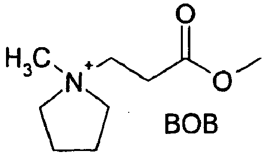 | > 5V |
| 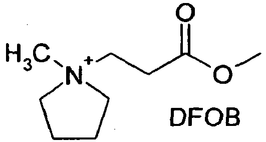 | > 5V |
| 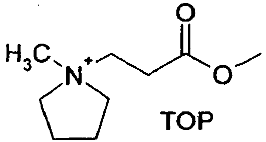 | > 5V |
| 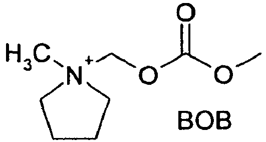 | > 5V |
| 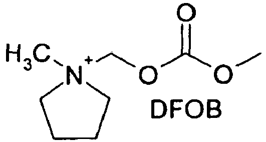 | > 5V |

| | |
|---|---|
| * Referenz = LiPF₆ in EC : DEC (3:7) | |

### Beispiel 11: Untersuchungen zur Oxidationsstabilität

In einer Messzelle mit Platinarbeitselektrode, Lithiumgegenelektrode und Lithiumreferenzelektrode werden jeweils 5 Zyklovoltammogramme hintereinander aufgenommen. Hierzu wird ausgehend vom Ruhepotential das Potential zuerst mit einer Vorschubgeschwindigkeit von 10 mV/s auf 6,0 V gegen Li/Li⁺ erhöht und im weiteren Verlauf zurück auf das Ruhepotential gefahren.

Als Elektrolyt wird 1M LiPF₆ in EC:DEC verwendet zu dem jeweils zwischen ca. 10% einer reaktiven Ionischen Flüssigkeit ausgewählt aus Tabelle 15 gegeben wird. Das Oxidationspotential wird zu > 5V gegen Li/Li⁺ bestimmt. In keinem der untersuchten Systeme wird ein Signal gefunden, das unter der Oxidationsstabilität des Referenzelektrolyten liegt.

**Tab. 18**

| Ionische Flüssigkeit | Oxidationstabilität Eₒₓ |
|---|---|
| Referenz* kein IL Additiv | > 5V |
| 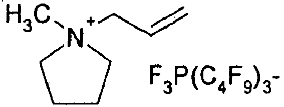 | > 5V |
| 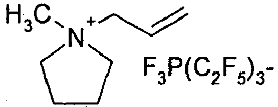 | > 5V |
| 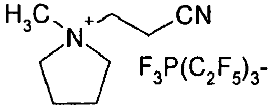 | > 5V |
| 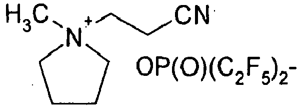 | > 5V |
| 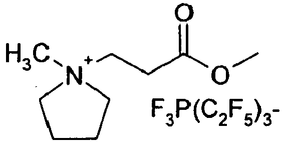 | > 5V |
| 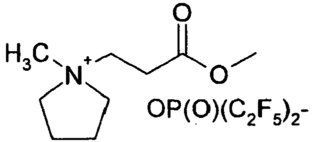 | > 5V |
| 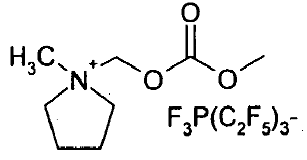 | > 5V |
| 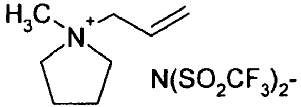 | > 5V |
| 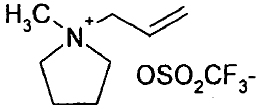 | 4,6 - 4,8V |
| 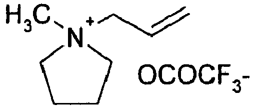 | 4,5- 4,7V |
| 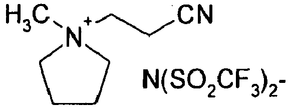 | > 5V |
| 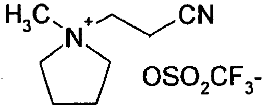 | 4,6 - 4,8V |
| 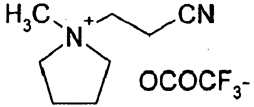 | 4,7- 4,8V |
| 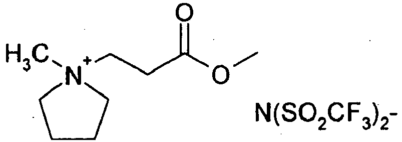 | > 5V |
| 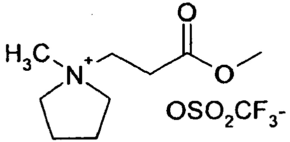 | 4,6 - 4,8V |
| 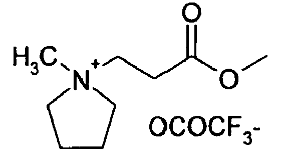 | 4,5- 4,7V |
| 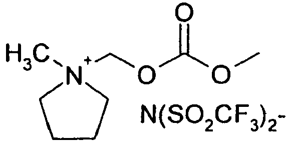 | > 5V |
| 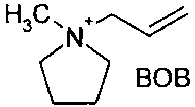 | 4,7- 4,8V |
| 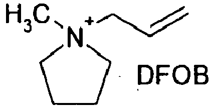 | 4,8- 5,0 V |
| 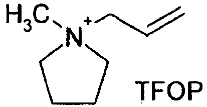 | 4,8- 5,0 V |
| 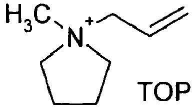 | 4,7- 4,8V |
| 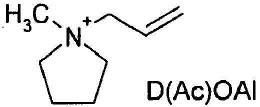 | > 5V |
| 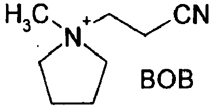 | 4,7- 4,8V |
| 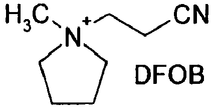 | 4,8- 5,0 V |
| 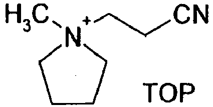 | 4,7- 4,8V |
| 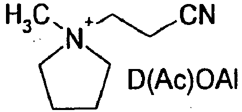 | > 5V |
| 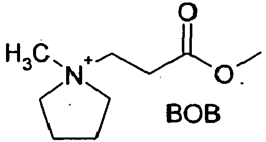 | 4,7- 4,8V |
| 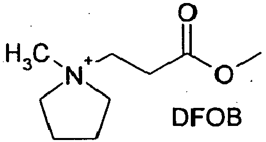 | 4,8- 5,0 V |
| 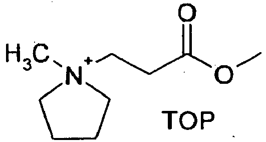 | 4,7- 4,8V |
| 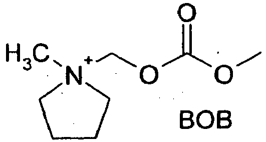 | 4,7- 4,8V |
| 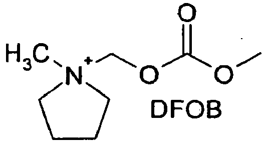 | 4,8- 5,0 V |

| | |
|---|---|
| * Referenz = LiPF₆ in EC : DEC (3:7) | |

## Patentansprüche

1. Ionische Flüssigkeiten der allgemeinen Formel I
K⁺ A⁻ (I)
worin bedeuten:
K⁺ ein Kation ausgewählt aus der Gruppe der allgemeinen Formeln II bis IX
NR₃-R5 PR₃-R5 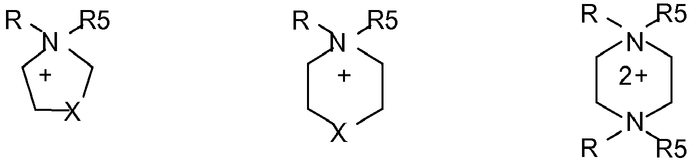 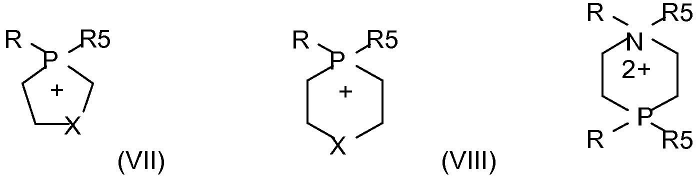
wobei
X CH₂, O, S oder NR'
R' -(CH₂)ₙ-CN, Alkyl C₁ bis C₁₆, R H, Alkyl C₁ bis C₁₆,
R5 -(CH₂)ₙ-O-C(O)-R, -(CH₂)ₙ-C(O)-OR, -(CH₂)ₙ-O-C(O)-OR, -(CH₂)ₙ-HC=CH-R oder -(CH₂)ₙ-CN, wobei einzelne der CH₂-Gruppen durch O, S oder NR ersetzt sein können mit n = 1 bis 8
und
A- ein Anion ausgewählt aus der Gruppe 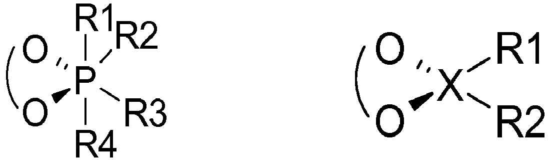
wobei 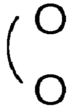 ein 1,2- oder 1,3- Diol, eine 1,2 -oder 1,3- Dicarbonsäure oder 1,2- oder 1,3-Hydroxycarbonsäure
X B oder Al,
R₁ bis R₄ Halogen, und/oder ein fluorierter oder nicht fluorierter Alkoxy- oder Carboxy-Rest
ist.

2. Ionische Flüssigkeiten nach Anspruch 1, **dadurch gekennzeichnet, dass** das Kation K⁺ eine Verbindung ausgewählt aus der allgemeinen Formel IV 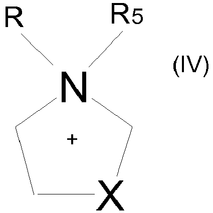 wobei
X CH₂, O, S oder NR'
R' -(CH₂)ₙ-CN, Alkyl C₁ bis C₁₆,R H, Alkyl C₁ bis C₁₆,
R₅ -(CH₂)ₙ-O-C(O)-R, -(CH₂)ₙ-C(O)-OR, -(CH₂)ₙ-O-C(O)-OR, -(CH₂)ₙ-HC=CH-R oder -(CH₂)ₙ-CN, wobei einzelne der CH₂-Gruppen durch O, S oder NR sein können, mit n = 1 bis 8 ist.

3. Elektrolyt enthaltend mindestens ein Leitsalz, ein aprotisches Lösungsmittel oder Lösungsmittelgemisch, mindestens eine ionische Flüssigkeit nach Anspruch 1 und/oder 2 sowie gegebenenfalls weitere Additive.

4. Elektrolyt nach Anspruch 3, **dadurch gekennzeichnet, dass** das Leitsalz ein Lithiumleitsalz wie LiPF₆, LiN(SO₂CF₃)₂, LiN(SO₂C₂F₅)₂, LiF₃P(C₂F₅)₃, LiF₃P(C₄F₉)₃, LiB(C₂O₄)₂ oder LiF₂B(C₂O₄)₂ ist.

5. Elektrolyt nach Anspruch 3, **dadurch gekennzeichnet, dass** das Leitsalz ausgewählt wird aus folgenden Verbindungen:
N(C₂H₅)₄BF₄, N(C₂H₅)₄PF₆, N(C₂H₅)₃(CH₃)BF₄, N(C₂H₅)₃(CH₃) PF₆,
N(C₂H₅)₄N(SO₂CF₃)₂, N(C₂H₅)₃(CH₃)N(SO₂CF₃)₂,
N(C₂H₅)₄F₃P(C₂F₅)₃, N(C₂H₅)₃(CH₃)F₃P(C₂F₅)₃

6. Elektrolyt nach einem oder mehreren der Ansprüche 3 bis 5, **dadurch gekennzeichnet, dass** das aprotische Lösungsmittel aus organischen offenkettigen oder zyklischen Carbonaten, Carbonsäureestern, Nitrilen, Ethern, Lactonen oder einem Gemisch daraus besteht.

7. Verfahren zur Herstellung von ionischen Flüssigkeiten der Formel I gemäß Anspruch 1 umfassend folgende Schritte:
- Herstellen von heterozyklischen Kationen K⁺ mit Alkylen-, Carboxylat-, Carbonat- oder Cyano-haltigen Seitenketten gemäß Anspruch 1 als Oniumchloride oderbromide aus den entsprechenden Aminen, Phosphinen, Halogencarboxylaten, Halogencarbonaten, Halogen-Alkyl- nitrilen oder Alkylhalogeniden nach herkömmlichen nasschemischen Methoden,
- Umsetzung dieser kationischen Oniumchloride oder -bromide mit den entsprechenden Lithium- oder Kalium-Boraten, -Phosphaten oder -Aluminaten im wässrigen und/oder alkoholischen Milieu oder organisches Lösungsmittels oder ohne Lösungsmittel.

8. Elektrochemische und/oder elektrooptische Vorrichtung enthaltend mindestens einen Elektrolyten, welcher mindestens eine ionische Flüssigkeit der allgemeinen Formel I nach Anspruch 1 enthält.

9. Elektrochemische und/oder elektrooptische Vorrichtung nach Anspruch 8, **dadurch gekennzeichnet, dass** es sich um mindestens eine Solarzelle, Lithiumionenbatterie, Lithiumbatterie, einen Doppelschicht - und Superkondensator, Lithium-Kondensator, eine Licht-emittierende Vorrichtung, einen elektrochemischen Sensor und/oder einen Biosensor handelt.

10. Verwendung von ionischen Flüssigkeiten der allgemeinen Formel I nach Anspruch 1 als Leitsalz oder Additiv in Elektrolyten für elektrochemische oder elektrooptische Zellen.

11. Verwendung von ionischen Flüssigkeiten der Formel I nach Anspruch 1 als Leitsalz oder Additiv in Elektrolyten für Batterien.

12. Verwendung von ionischen Flüssigkeiten der Formel I nach Anspruch 1 als Leitsalz oder Additiv in Elektrolyten in sekundären Lithiumbatterien.

13. Verwendung von ionischen Flüssigkeiten der Formel I nach Anspruch 1 in Doppelschicht- und Superkondensatoren oder Lithium-Kondensatoren.

14. Verwendung von Elektrolyten nach einem oder mehreren der Ansprüche 3 bis 6 in elektrochemischen und/oder elektrooptischen Vorrichtungen.

15. Verwendung von Elektrolyten nach einem oder mehreren der Ansprüche 3 bis 6 in Lithium- oder Lithiumionen-Batterien.

16. Verwendung von Elektrolyten nach einem oder mehreren der Ansprüche 3 bis 6 in Doppelschicht- und Superkondensatoren oder Lithium-Kondensatoren.
